# EUROPÄISCHE PATENTSCHRIFT

(11) **EP 0 906 270 B1**
(45) Veröffentlichungstag und Bekanntmachung des Hinweises auf die Patenterteilung: **25.10.2000**
(21) Anmeldenummer: 97923870.6
(22) Anmeldetag: 12.05.1997
(51) Int. Cl.: C07C 253/30, C07D 231/16, C07D 239/54

(54) **VERFAHREN ZUR HERSTELLUNG VON HYDROXYARENEN**
PROCESS FOR PRODUCING HYDROXYARENES
PROCEDE DE PREPARATION D'HYDROXYARENES

(30) Priorität: 24.05.1996 DE 19620992
(43) Veröffentlichungstag der Anmeldung: 07.04.1999
(73) Patentinhaber: BAYER AG, 51368 Leverkusen (DE)
(72) Erfinder: LINKER, Karl-Heinz, D-51377 Leverkusen (DE); HAAS, Wilhelm, D-50259 Pulheim (DE); SCHALLNER, Otto, D-40789 Monheim (DE); FINDEISEN, Kurt, D-51375 Leverkusen (DE); ANDREE, Roland, D-40764 Langenfeld (DE); DREWES, Mark, Wilhelm, D-40764 Langenfeld (DE)
(86) Internationale Anmeldenummer: EP9702410
(87) Internationale Veröffentlichungsnummer: WO9745398

(56) Entgegenhaltungen:
- DE-A- 3 835 168

## Beschreibung

Die Erfindung betrifft ein neues Verfahren zur Herstellung von Hydroxyarenen, welche als Wirkstoffe oder Zwischenprodukte zur Herstellung von Wirkstoffen, insbesondere von Herbiziden, verwendet werden können.

Herstellungsmethoden für Hydroxyarene ("Phenole") sind lange bekannt und gehören zum "Lehrbuchwissen" der organischen Chemie. Im allgemeinen werden Hydroxyarene durch Umsetzung von Arenen, welche leicht nucleophil substituierbare Gruppen enthalten, mit Metallhydroxiden hergestellt. Probleme können hierbei auftreten, wenn die Ausgangsstoffe weitere leicht nucleophil angreifbare Gruppen, wie z.B. Cyano- oder Ester-gruppierungen enthalten. Bei der alternativ möglichen Synthese durch Spaltung von entsprechenden Methoxyarenen (vgl. DE-A 38 35 168 und Bull. Korean Chem. Soc. 14 (1993), 717) sind ebenfalls unerwünschte Nebenreaktionen zu beobachten. Die Aufgabe der vorliegenden Erfindung bestand darin, eine Verfahrensmethode zu finden, die es ermöglicht, Hydroxygruppen in Arene einzuführen, ohne daß andere leicht angreifbare Gruppen, wie insbesondere die Cyanogruppe, verändert werden.

Die Aufgabe wurde dadurch gelöst, daß man Hydroxyarene der allgemeinen Formel (I) 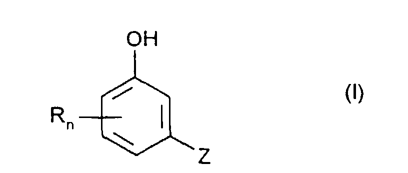 in welcher
- n: für die Zahlen 1, 2, 3 oder 4 steht,
- R: für Cyano, Carboxy, Formyl, Nitro, Halogen oder für jeweils gegebenenfalls substituiertes Alkyl, Alkoxy, Alkylthio, Alkylsulfinyl, Alkylsulfonyl, Alkylcarbonyl oder Alkoxycarbonyl steht und
- Z: für Wasserstoff, Cyano, Nitro, Halogen, Alkyl, Halogenalkyl, oder für jeweils gegebenenfalls substituiertes, monocyclisches oder bicyclisches, gesättigtes oder ungesättigtes Heterocyclyl, Heterocyclylamino oder Heterocyclylimino steht,
in hohen Ausbeuten und in guter Qualität erhält,
wenn man Halogenarene der allgemeinen Formel (II) 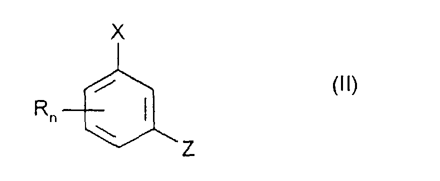 in welcher
- n, R und Z: die oben angegebenen Bedeutungen haben und
- x: für Halogen steht,
mit 3-Hydroxy-propionitril der Formel (III)

HO-CH₂CH₂-CN (III)

gegebenenfalls in Gegenwart eines Reaktionshilfsmittels und gegebenenfalls in Gegenwart eines Verdünnungsmittels bei Temperaturen zwischen 0°C und 100°C umsetzt.

Überraschenderweise können nach dem erfindungsgemäßen Verfahren Hydroxyarene der allgemeinen Formel (I) auf einfache und schonende Weise in hohen Ausbeuten und in guter Qualität erhalten werden, wobei gegenüber Nucleophilen empfindliche Gruppen, wie insbesondere Cyanogruppen, unbehelligt bleiben.

Das erfindungsgemäße Verfahren stellt somit eine wertvolle Bereicherung des Standes der Technik dar.

Das erfindungsgemäße Verfahren betrifft vorzugsweise die Herstellung von Verbindungen der Formel (I), in welcher
- n: für die Zahlen 1, 2 oder 3 steht,
- R: für Cyano, Carboxy, Formyl, Nitro, Halogen oder für jeweils gegebenenfalls durch Cyano, Halogen, C₁-C₄-Alkoxy oder C₁-C₄-Alkylthio substituiertes Alkyl, Alkoxy, Alkylthio, Alkylsulfinyl, Alkylsulfonyl, Alkylcarbonyl oder Alkoxycarbonyl mit jeweils 1 bis 6 Kohlenstoffatomen steht und
- Z: für Wasserstoff, Cyano, Halogen oder für jeweils gegebenenfalls substituiertes, monocyclisches ode bicyclisches, gesättigtes oder ungesättigtes Heterocyclyl, Heterocyclylamino oder Heterocyclylimino mit jeweils 2 bis 6 Kohlenstoffatomen und 1 bis 4 Stickstoffatomen im heterocyclischen Ringsystem steht, welches gegebenenfalls zusätzlich ein Sauerstoff- oder Schwefelatom und/oder gegebenenfalls bis zu drei Gruppierungen aus der Reihe -CO-, -CS-, -SO- und/oder SO₂- enthält, und welches gegebenenfalls substituiert ist durch eine oder mehrere Gruppierungen aus der Reihe Nitro, Hydroxy, Amino, Cyano, Carboxy, Carbamoyl, Thiocarbamoyl, Halogen, C₁-C₆-Alkyl (welches gegebenenfalls durch Halogen oder C₁-C₄-Alkoxy substituiert ist), C₂-C₆-Alkenyi oder C₂-C₆-Alkinyl (welche jeweils gegebenenfalls durch Halogen substituiert sind), C₁-C₆-Alkoxy oder C₁-C₆-Alkoxy-carbonyl (welche jeweils gegebenenfalls durch Halogen oder C₁-C₄-Alkoxy substituiert sind), C₂-C₆-Alkenyloxy oder C₂-C₆-Alkinyloxy (welche jeweils gegebenenfalls durch Halogen substituiert sind), C₁-C₆-Alkylthio, C₂-C₆-Alkenylthio oder C₂-C₆-Alkinylthio (welche jeweils gegebenenfalls durch Halogen substituiert sind), C₁-C₆-Alkylamino oder Di-(C₁-C₄-alkyl)-amino, C₃-C₆-Cycloalkyl oder C₃-C₆-Cycloalkyl-C₁-C₄-alkyl (welche jeweils gegebenenfalls durch Halogen und/oder C₁-C₄-Alkyl substituiert sind), Phenyl, Phenoxy, Phenylthio, Phenylsulfinyl, Phenylsulfonyl oder Phenylamino (welche jeweils gegebenenfalls durch Nitro, Cyano, Halogen, C₁-C₄-Alkyl, C₁-C₄-Halogenalkyl, C₁-C₄-Alkyloxy, C₁-C₄-Halogenalkyloxy und/oder C₁-C₄-Alkoxy-carbonyl substituiert sind).

Das erfindungsgemäße Verfahren betrifft insbesondere die Herstellung von Verbindungen der Formel (I), in welcher
- n: für die Zahlen 1 oder 2 steht,
- R: für Cyano, Carboxy, Formyl, Nitro, Halogen oder für jeweils gegebenenfalls durch Cyano, Halogen, Methoxy, Ethoxy, Methylthio oder Ethylthio substituiertes Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl, Ethylsulfonyl, Acetyl, Propionyl, Methoxycarbonyl oder Ethoxycarbonyl steht und
- Z: für Wasserstoff, Cyano, Halogen oder für jeweils gegebenenfalls substituiertes, monocyclisches oder bicyclisches, gesättigtes oder ungesättigtes Heterocyclyl, Heterocyclylamino oder Heterocyclylimino mit jeweils 2 bis 6 Kohlenstoffatomen und 1 bis 4 Stickstoffatomen im heterocyclischen Ringsystem steht, welches gegebenenfalls zusätzlich ein Sauerstoff- oder Schwefelatom und/oder gegebenenfalls bis zu zwei Gruppierungen aus der Reihe -CO-, -CS-, -SO- und/oder SO₂- enthält, und welches gegebenenfalls substituiert ist durch eine oder mehrere Gruppierungen aus der Reihe Nitro, Hydroxy, Amino, Cyano, Carboxy, Carbamoyl, Thiocarbamoyl, Fluor, Chlor, Brom; Methyl, Ethyl, n- oder i-Propyl, n-, i-, s- oder t-Butyl, (welche gegebenenfalls durch Fluor, Chlor, Methoxy oder Ethoxy substituiert sind); Propenyl, Butenyl, Propinyl oder Butinyl (welche jeweils gegebenenfalls durch Fluor oder Chlor substituiert sind); Methoxy, Ethoxy, n-oder i-Propoxy, n-, i-, s- oder t-Butoxy, Methoxycarbonyl oder Ethoxycarbonyl (welche jeweils gegebenenfalls durch Fluor, Chlor, Methoxy oder Ethoxy substituiert sind); Propenyloxy, Butenyloxy, Propinyloxy oder Butinyloxy (welche gegebenenfalls durch Fluor oder Chlor substituiert sind); Methylthio, Ethylthio, n- oder i-Propylthio, n-, i-, s- oder t-Butylthio, Propenylthio, Butenylthio, Propinylthio oder Butinylthio (welche jeweils gegebenenfalls durch Fluor oder Chlor substituiert sind); Methylamino, Ethylamino, n- oder i-Propylamino, n-, i-, s- oder t-Butylamino, Dimethylamino oder Diethylamino; Cyclopropyl, Cyclobutyl, Cyclopentyl, Cyclohexyl, Cyclopropylmethyl, Cyclobutylmethyl, Cyclopentylmethyl oder Cyclohexylmethyl (welche jeweils gegebenenfalls durch Fluor, Chlor, Methyl, Ethyl, n- oder i-Propyl substituiert sind), Phenyl, Phenoxy, Phenylthio, Phenylsulfinyl, Phenylsulfonyl oder Phenylamino (welche jeweils gegebenenfalls durch Nitro, Cyano, Fluor, Chlor, Brom, Methyl, Ethyl, n-oder i-Propyl, Trifluormethyl, Methoxy, Ethoxy, n- oder i-Propoxy, Difluormethoxy, Trifluormethoxy, Methoxycarbonyl oder Ethoxycarbonyl substituiert sind).
- Z: steht in den Formeln (I) und (II) insbesondere für die nachstehend aufgeführten heterocyclischen Gruppierungen: 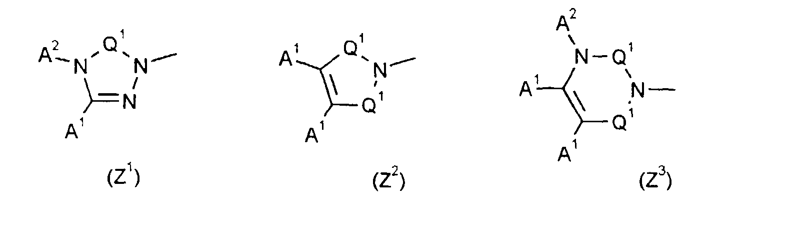 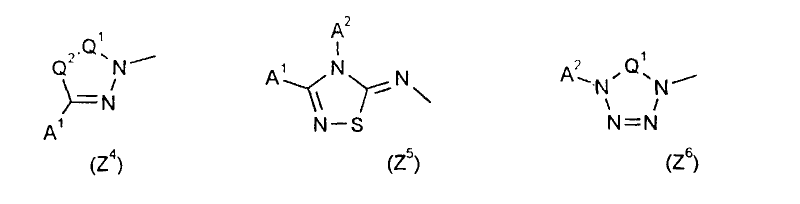 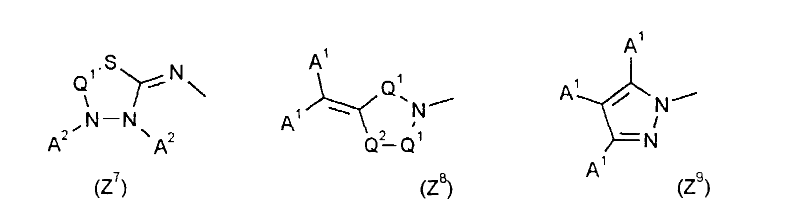 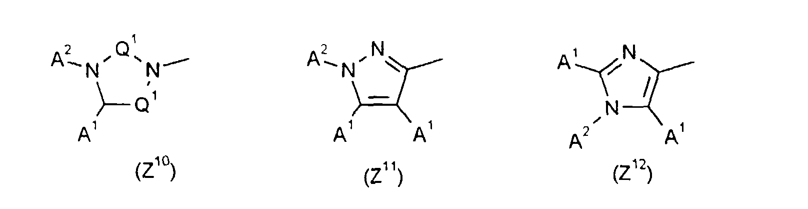 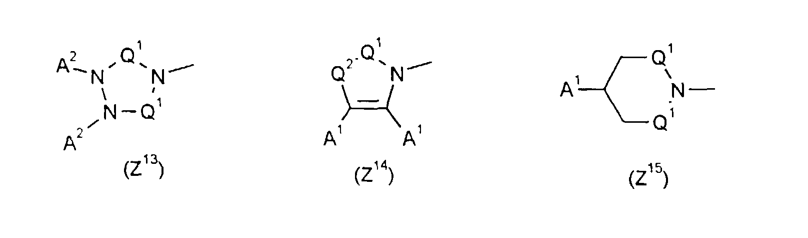 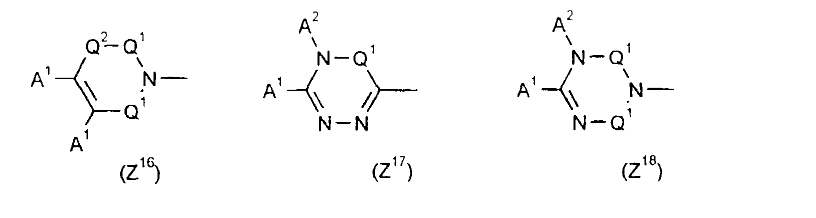 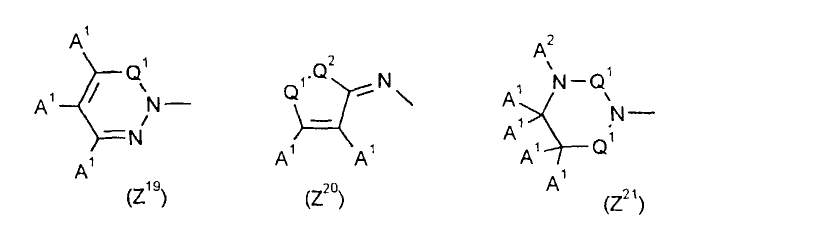 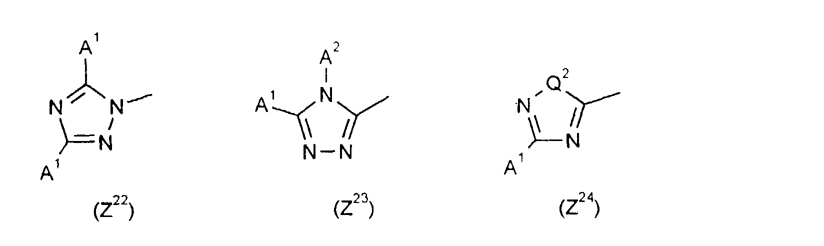 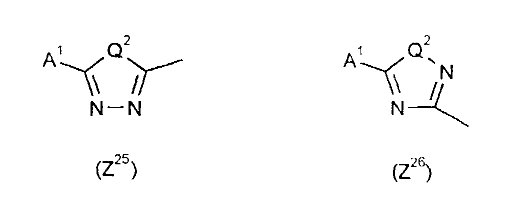 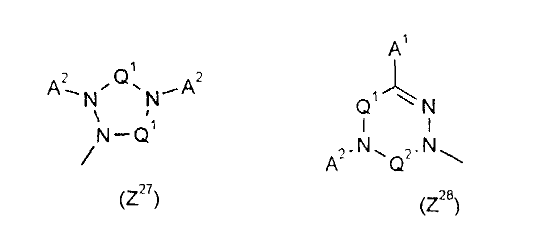
wobei jeweils
- Q¹: für eine Gruppierung aus der Reihe -CO-, -CS-, -CH₂-, -CH(OH)-, -CHCl-, -CHBr-, -C(=CH₂)-, -C(=(CHF)-, -C(=CF₂)-, -C(=CHCl)-, -C(=CHBr)-, -C(=CHOCHF₂)-, -C(=CHOCF₃)-, -C(-CHOCH₂CF₃)- steht,
- Q²: für Sauerstoff, Schwefel oder eine Gruppierung aus der Reihe -CO-, -CS-, -CH₂-, -CHF-, -CF₂-, -CHCl-, -CHBr-, -CHOCHF₂-, -CHOCF₃-, -CHOCH₂CF₃- steht,
- A¹: für Wasserstoff, Amino, Nitro, Cyano, Carboxy, Carbamoyl, Fluor, Chlor, Brom, Methyl, Ethyl, n- oder i-Propyl, Cyclopropyl, Difluormethyl, Trifluormethyl, Chlordifluormethyl, Methoxy, Ethoxy, n- oder i-Propoxy, Difluormethoxy, Trifluormethoxy, Chlordifluormethoxy, Methylthio, Ethylthio, n- oder i-Propylthio, Difluormethylthio, Trifluormethylthio, Chlordifluormethylthio, Methylamino, Ethylamino, n- oder i-Propylamino, Dimethylamino, Diethylamino, Methoxycarbonyl oder Ethoxycarbonyl steht, und
- A²: für Wasserstoff, Hydroxy, Amino, Cyano, Methyl, Ethyl, n- oder i-Propyl, Difluormethyl, Methoxy, Ethoxy, n- oder i-Propoxy steht,
oder wobei gegebenenfalls zwei benachbarte Gruppen - A¹ und A¹ oder A² und A² oder A¹ und A² - zusammen für jeweils gegebenenfalls durch Fluor, Chlor, Brom, Methyl, Ethyl, n- oder i-Propyl substituiertes und gegebenenfalls durch Sauerstoff, Schwefel oder eine Gruppierung aus der Reihe -SO-, SO₂-, -N(CH₃)-oder N(C₂H₅)- am Anfang (bzw. am Ende) oder innerhalb der Kohlenwasserstoffkette unterbrochenes Alkandiyl oder Alkendiyl mit jeweils bis zu 4 Kohlenstoffatomen stehen.

Beispiele für die nach dem erfindungsgemäßen Verfahren herzustellenden Verbindungen der Formel (I) sind in den nachstehenden Gruppen aufgeführt.

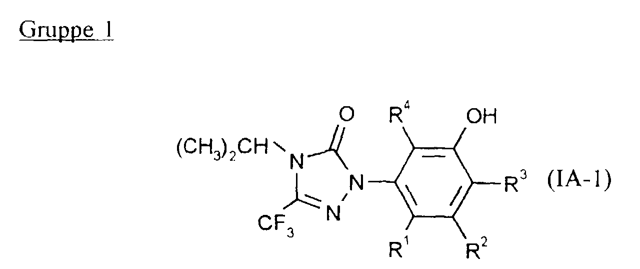

| | | | |
|---|---|---|---|
| R¹ | R² | R³ | R⁴ |
| F | H | CN | H |
| Cl | H | CN | H |
| H | Cl | H | H |
| Cl | H | Cl | F |
| F | H | Cl | H |
| F | Cl | Cl | H |
| Cl | H | CF₃ | Cl |
| CH₃ | H | CF₃ | Cl |
| Br | H | CF₃ | Cl |
| CN | H | CF₃ | Cl |
| SC₂H₅ | H | CN | H |
| OCH₃ | H | CN | H |
| F | Cl | CN | H |
| F | H | CF₃ | Cl |
| Cl | H | NO₂ | H |
| Cl | H | NO₂ | Cl |
| CF₃ | H | NO₂ | H |
| NO₂ | H | CF₃ | H |
| H | Cl | CN | CH₃ |

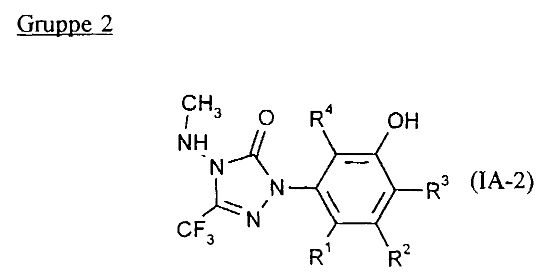

R¹, R², R³ und R⁴ haben hierbei beispielhaft die oben in Gruppe 1 angegebenen Bedeutungen. 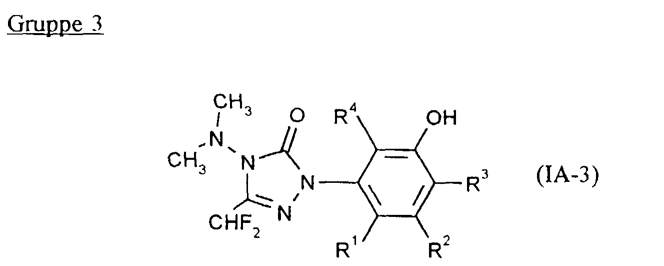 R¹, R², R³ und R⁴ haben hierbei beispielhaft die oben in Gruppe 1 angegebenen Bedeutungen. 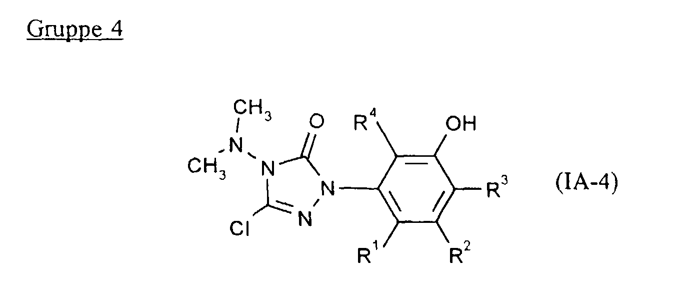 R¹, R², R³ und R⁴ haben hierbei beispielhaft die oben in Gruppe 1 angegebenen Bedeutungen. 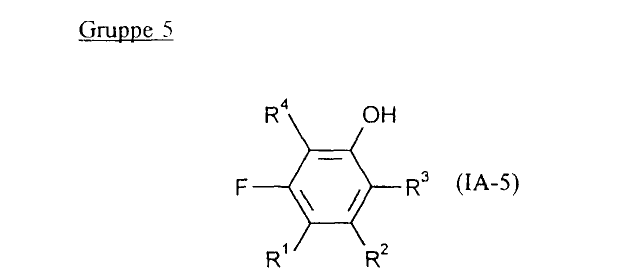 R¹, R², R³ und R⁴ haben hierbei beispielhaft die oben in Gruppe 1 angegebenen Bedeutungen. 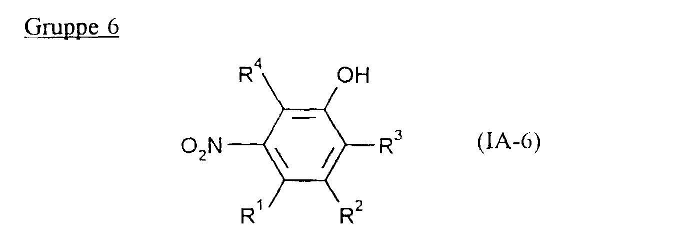 R¹, R², R³ und R⁴ haben hierbei beispielhaft die oben in Gruppe 1 angegebenen Bedeutungen. 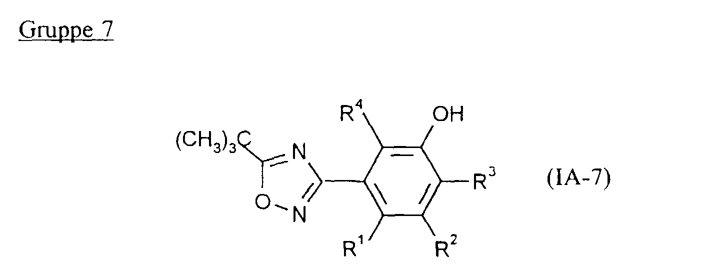 R¹, R², R³ und R⁴ haben hierbei beispielhaft die oben in Gruppe 1 angegebenen Bedeutungen. 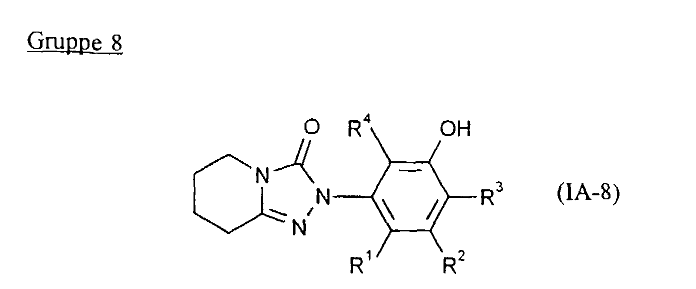 R¹, R², R³ und R⁴ haben hierbei beispielhaft die oben in Gruppe 1 angegebenen Bedeutungen. 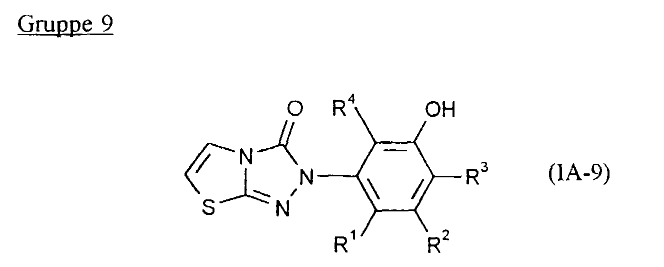 R¹, R², R³ und R⁴ haben hierbei beispielhaft die oben in Gruppe 1 angegebenen Bedeutungen. 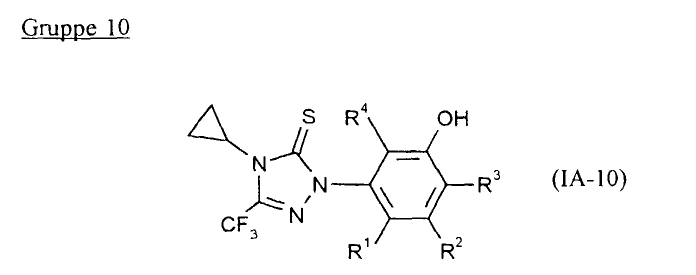 R¹, R², R³ und R⁴ haben hierbei beispielhaft die oben in Gruppe 1 angegebenen Bedeutungen. 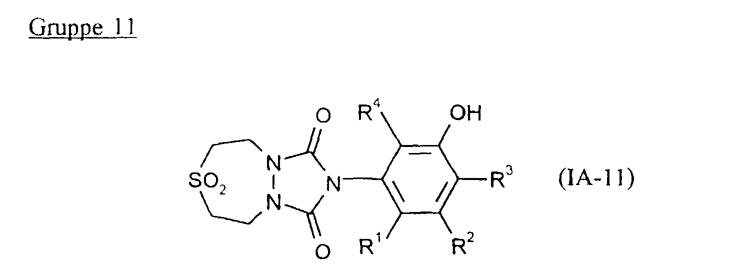 R¹, R², R³ und R⁴ haben hierbei beispielhaft die oben in Gruppe 1 angegebenen Bedeutungen. 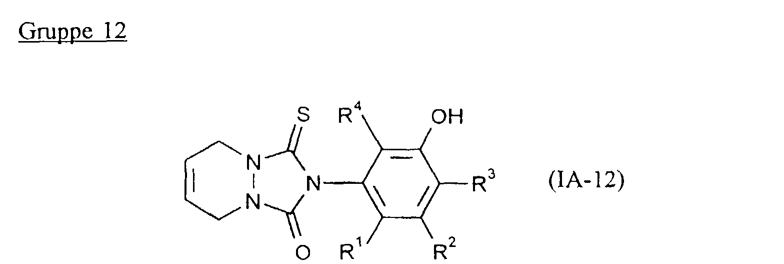 R¹, R², R³ und R⁴ haben hierbei beispielhaft die oben in Gruppe 1 angegebenen Bedeutungen. 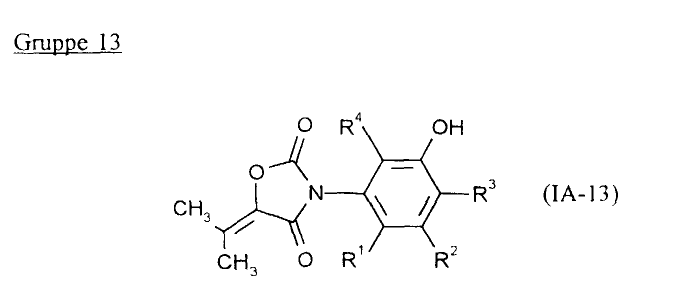 R¹, R², R³ und R⁴ haben hierbei beispielhaft die oben in Gruppe 1 angegebenen Bedeutungen. 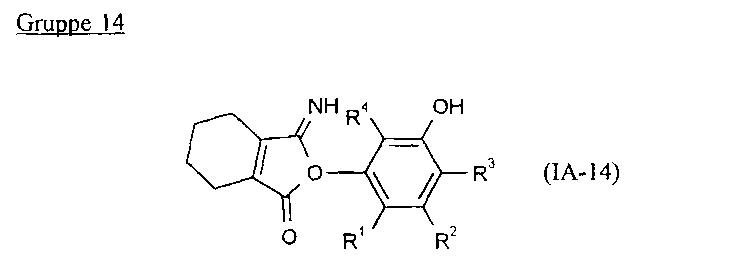 R¹, R², R³ und R⁴ haben hierbei beispielhaft die oben in Gruppe 1 angegebenen Bedeutungen. 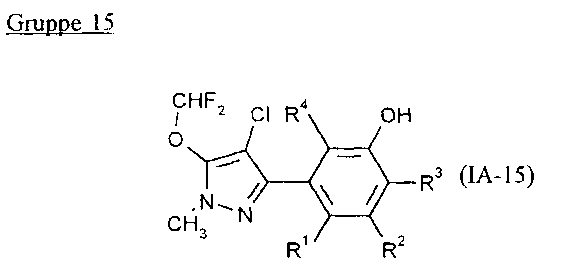 R¹, R², R³ und R⁴ haben hierbei beispielhaft die oben in Gruppe 1 angegebenen Bedeutungen. 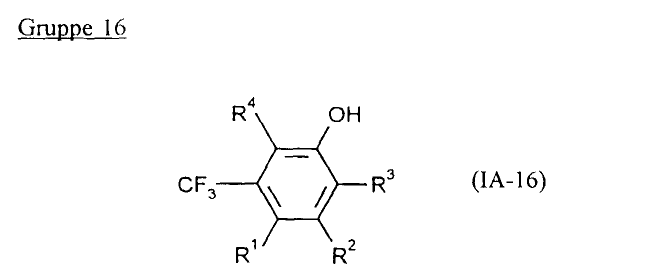 R¹, R², R³ und R⁴ haben hierbei beispielhaft die oben in Gruppe 1 angegebenen Bedeutungen. 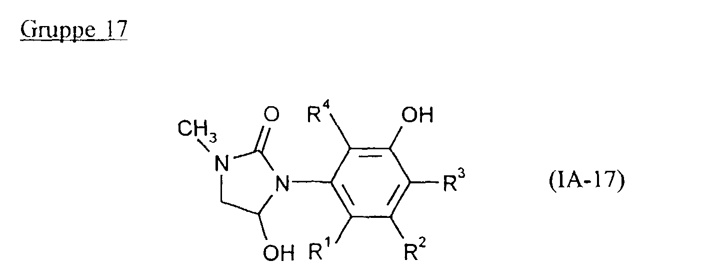 R¹, R², R³ und R⁴ haben hierbei beispielhaft die oben in Gruppe 1 angegebenen Bedeutungen. 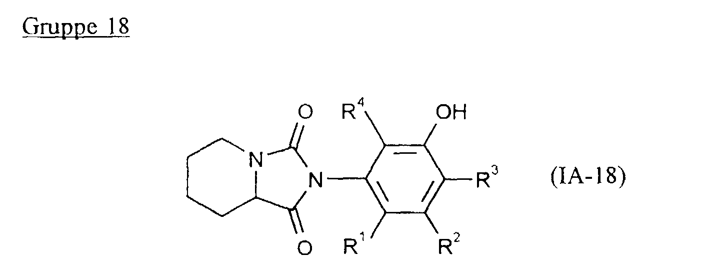 R¹, R², R³ und R⁴ haben hierbei beispielhaft die oben in Gruppe 1 angegebenen Bedeutungen. 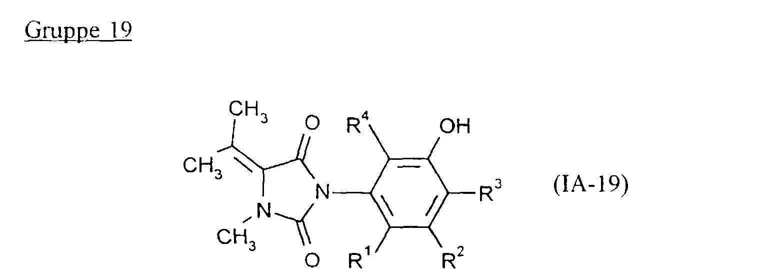 R¹, R², R³ und R⁴ haben hierbei beispielhaft die oben in Gruppe 1 angegebenen Bedeutungen. 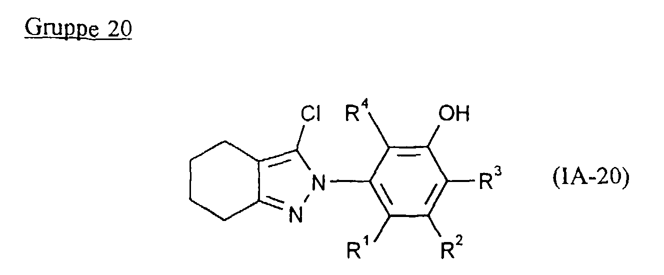 R¹, R², R³ und R⁴ haben hierbei beispielhaft die oben in Gruppe 1 angegebenen Bedeutungen. 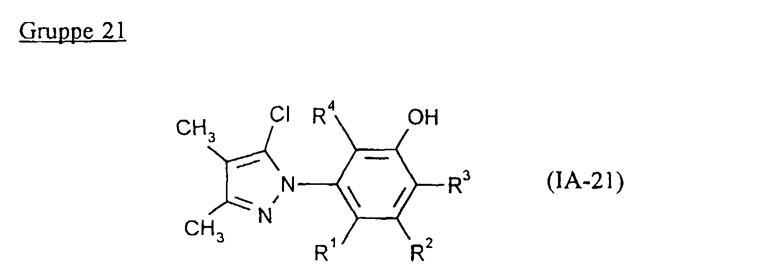 R¹, R², R³ und R⁴ haben hierbei beispielhaft die oben in Gruppe 1 angegebenen Bedeutungen. 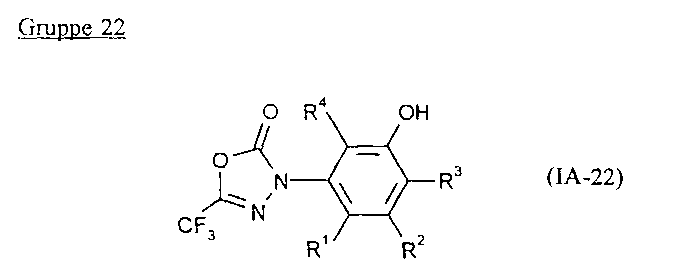 R¹, R², R³ und R⁴ haben hierbei beispielhaft die oben in Gruppe 1 angegebenen Bedeutungen. 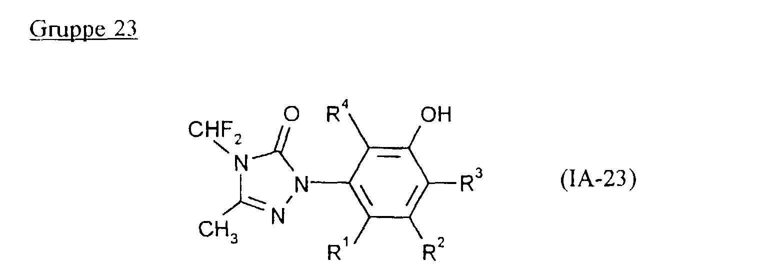 R¹, R², R³ und R⁴ haben hierbei beispielhaft die oben in Gruppe 1 angegebenen Bedeutungen. 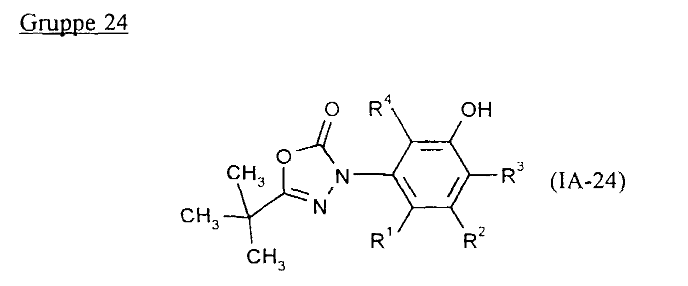 R¹, R², R³ und R⁴ haben hierbei beispielhaft die oben in Gruppe 1 angegebenen Bedeutungen. 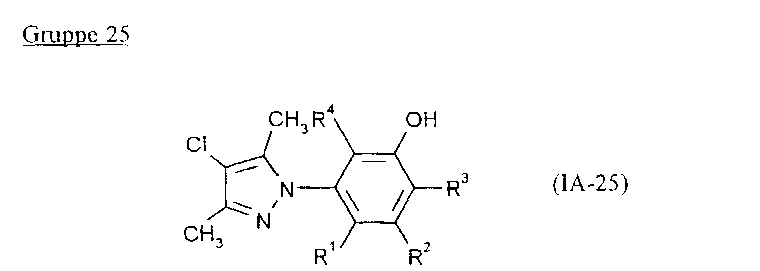 R¹, R², R³ und R⁴ haben hierbei beispielhaft die oben in Gruppe 1 angegebenen Bedeutungen. 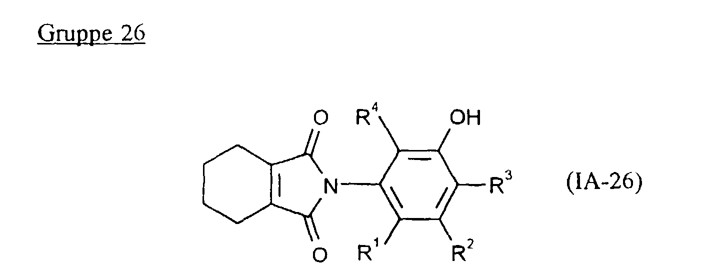 R¹, R², R³ und R⁴ haben hierbei beispielhaft die oben in Gruppe 1 angegebenen Bedeutungen. 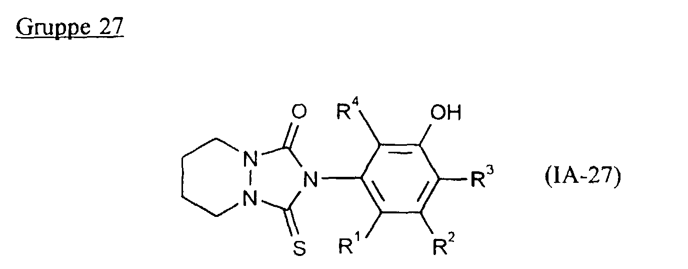 R¹, R², R³ und R⁴ haben hierbei beispielhaft die oben in Gruppe 1 angegebenen Bedeutungen. 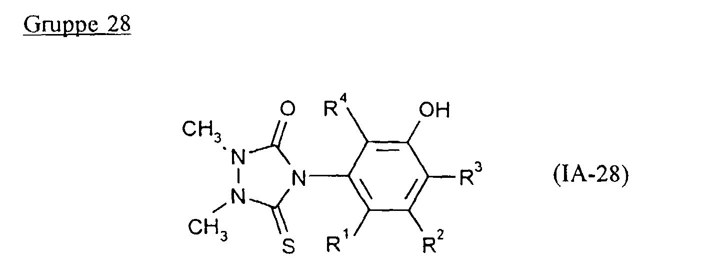 R¹, R², R³ und R⁴ haben hierbei beispielhaft die oben in Gruppe 1 angegebenen Bedeutungen. 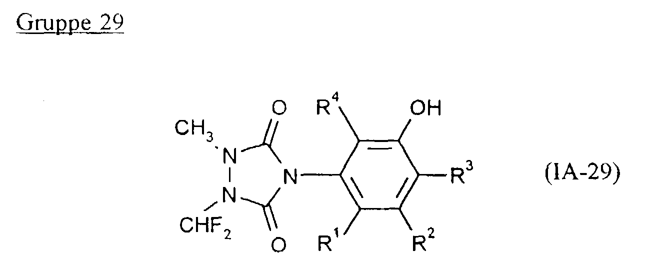 R¹, R², R³ und R⁴ haben hierbei beispielhaft die oben in Gruppe 1 angegebenen Bedeutungen. 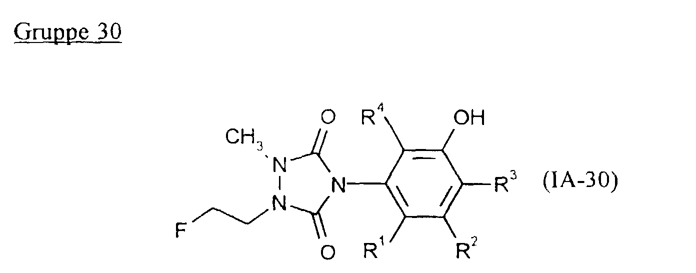 R¹, R², R³ und R⁴ haben hierbei beispielhaft die oben in Gruppe 1 angegebenen Bedeutungen. 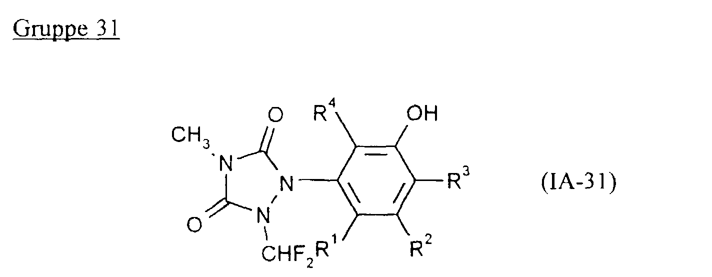 R¹, R², R³ und R⁴ haben hierbei beispielhaft die oben in Gruppe 1 angegebenen Bedeutungen. 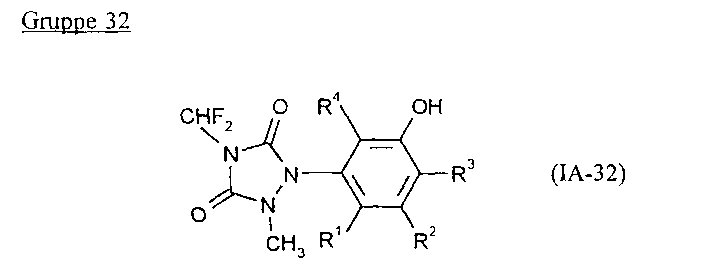 R¹, R², R³ und R⁴ haben hierbei beispielhaft die oben in Gruppe 1 angegebenen Bedeutungen. 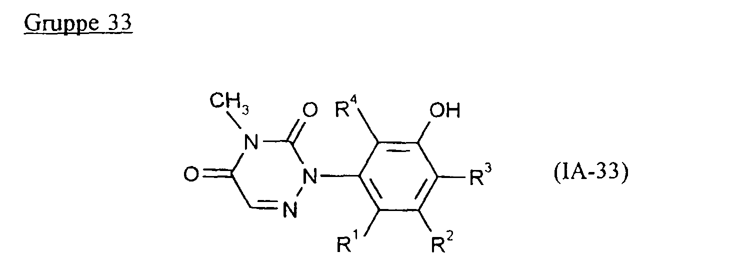 R¹, R², R³ und R⁴ haben hierbei beispielhaft die oben in Gruppe 1 angegebenen Bedeutungen. 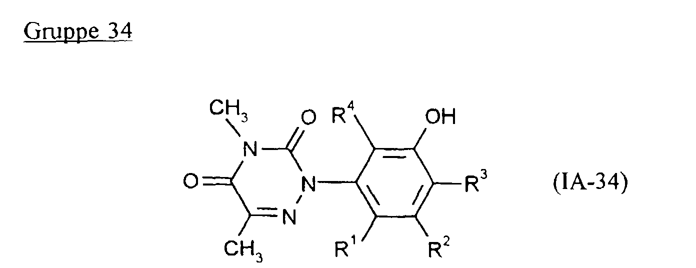 R¹, R², R³ und R⁴ haben hierbei beispielhaft die oben in Gruppe 1 angegebenen Bedeutungen. 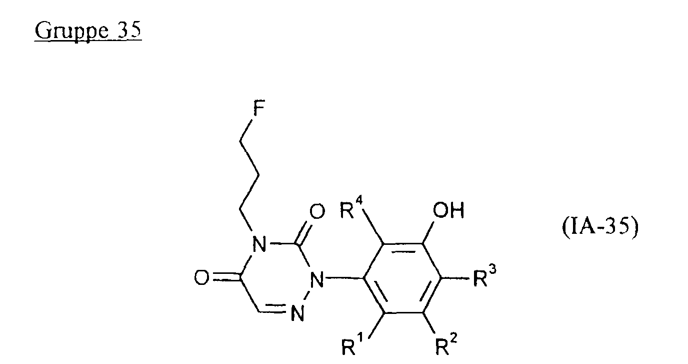 R¹, R², R³ und R⁴ haben hierbei beispielhaft die oben in Gruppe 1 angegebenen Bedeutungen. 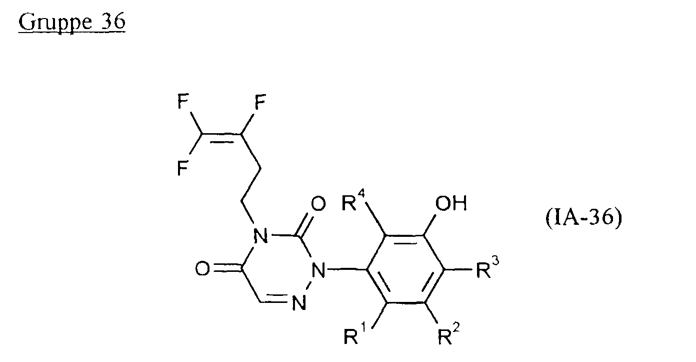 R¹, R², R³ und R⁴ haben hierbei beispielhaft die oben in Gruppe 1 angegebenen Bedeutungen. 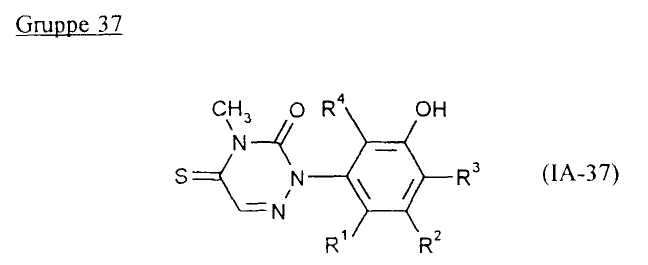 R¹, R², R³ und R⁴ haben hierbei beispielhaft die oben in Gruppe 1 angegebenen Bedeutungen. 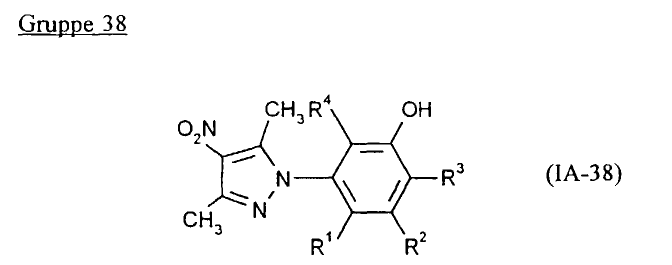 R¹, R², R³ und R⁴ haben hierbei beispielhaft die oben in Gruppe 1 angegebenen Bedeutungen. 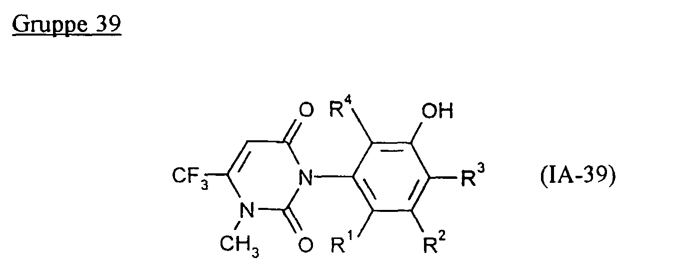 R¹, R², R³ und R⁴ haben hierbei beispielhaft die oben in Gruppe 1 angegebenen Bedeutungen. 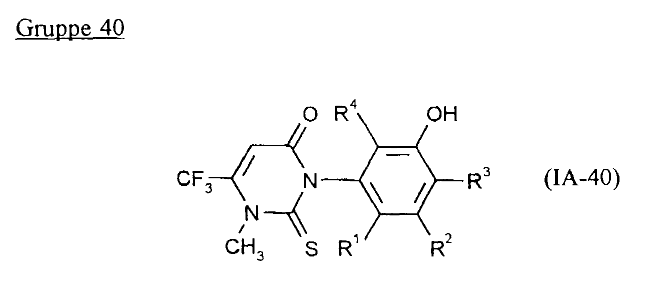 R¹, R², R³ und R⁴ haben hierbei beispielhaft die oben in Gruppe 1 angegebenen Bedeutungen. 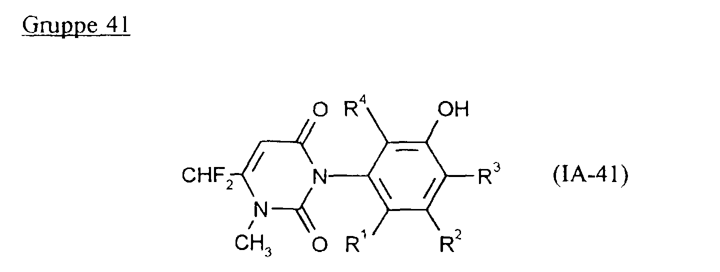 R¹, R², R³ und R⁴ haben hierbei beispielhaft die oben in Gruppe 1 angegebenen Bedeutungen. 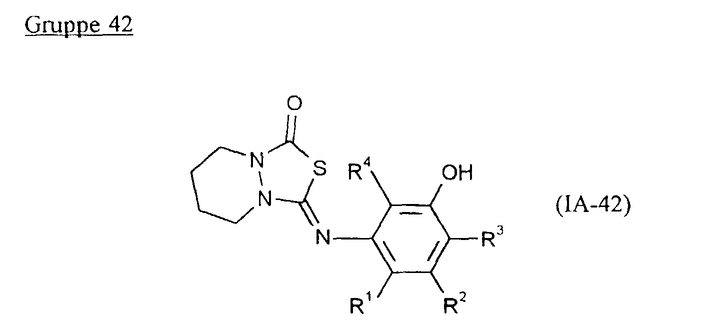 R¹, R², R³ und R⁴ haben hierbei beispielhaft die oben in Gruppe 1 angegebenen Bedeutungen. 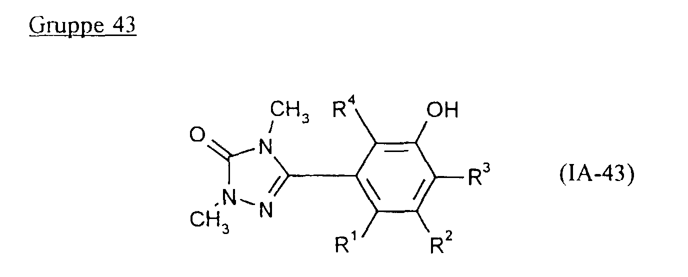 R¹, R², R³ und R⁴ haben hierbei beispielhaft die oben in Gruppe 1 angegebenen Bedeutungen. 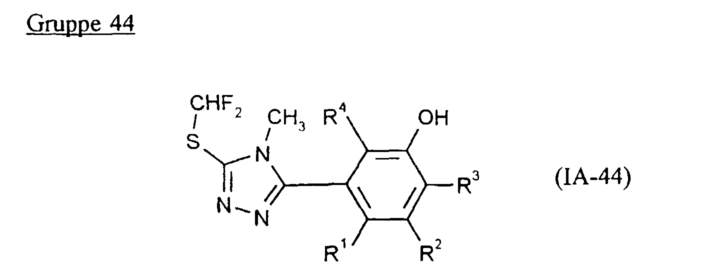 R¹, R², R³ und R⁴ haben hierbei beispielhaft die oben in Gruppe 1 angegebenen Bedeutungen. 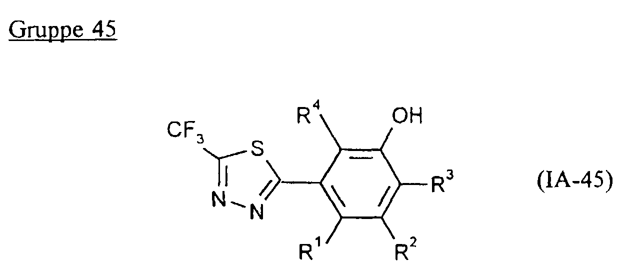 R¹, R², R³ und R⁴ haben hierbei beispielhaft die oben in Gruppe 1 angegebenen Bedeutungen. 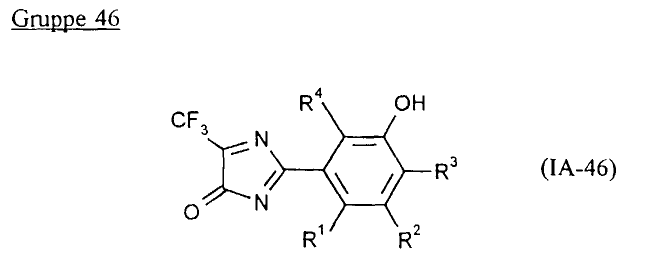 R¹, R², R³ und R⁴ haben hierbei beispielhaft die oben in Gruppe 1 angegebenen Bedeutungen. 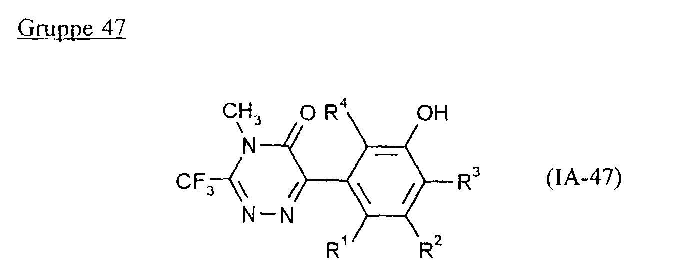 R¹, R², R³ und R⁴ haben hierbei beispielhaft die oben in Gruppe 1 angegebenen Bedeutungen. 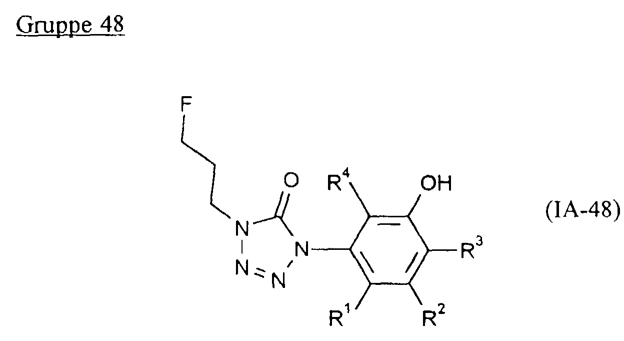 R¹, R², R³ und R⁴ haben hierbei beispielhaft die oben in Gruppe 1 angegebenen Bedeutungen. 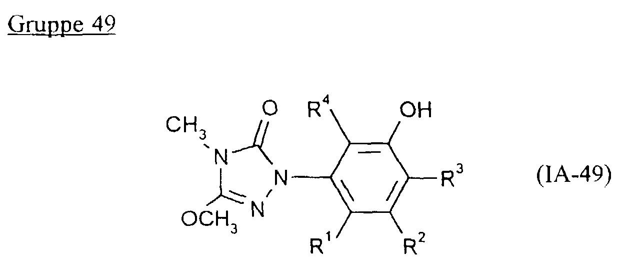 R¹, R², R³ und R⁴ haben hierbei beispielhaft die oben in Gruppe 1 angegebenen Bedeutungen 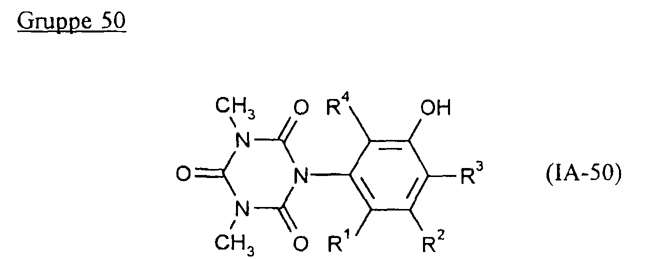 R¹, R², R³ und R⁴ haben hierbei beispielhaft die oben in Gruppe 1 angegebenen Bedeutungen. 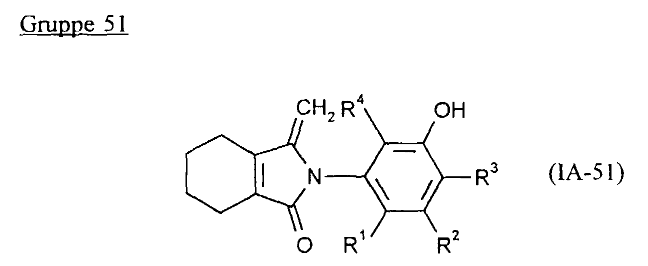 R¹, R², R³ und R⁴ haben hierbei beispielhaft die oben in Gruppe 1 angegebenen Bedeutungen. 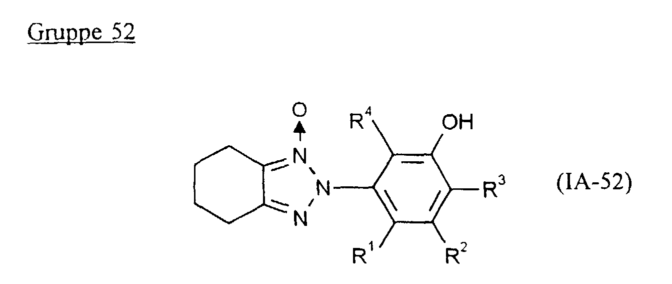 R¹, R², R³ und R⁴ haben hierbei beispielhaft die oben in Gruppe 1 angegebenen Bedeutungen. 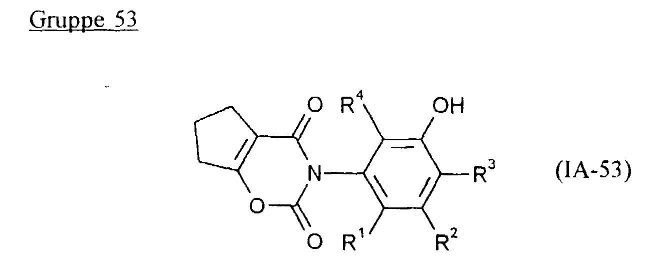 R¹, R², R³ und R⁴ haben hierbei beispielhaft die oben in Gruppe 1 angegebenen Bedeutungen. 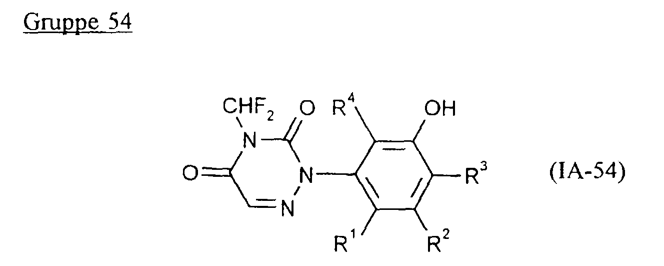 R¹, R², R³ und R⁴ haben hierbei beispielhaft die oben in Gruppe 1 angegebenen Bedeutungen. 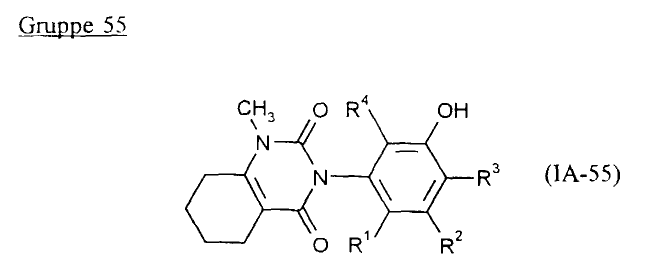 R¹, R², R³ und R⁴ haben hierbei beispielhaft die oben in Gruppe 1 angegebenen Bedeutungen. 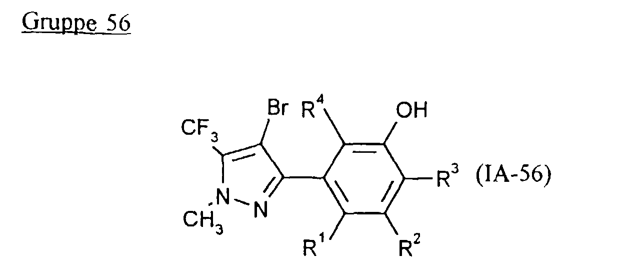 R¹, R², R³ und R⁴ haben hierbei beispielhaft die oben in Gruppe 1 angegebenen Bedeutungen. 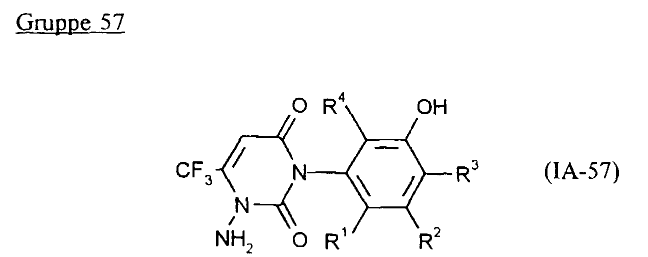 R¹, R², R³ und R⁴ haben hierbei beispielhaft die oben in Gruppe 1 angegebenen Bedeutungen. 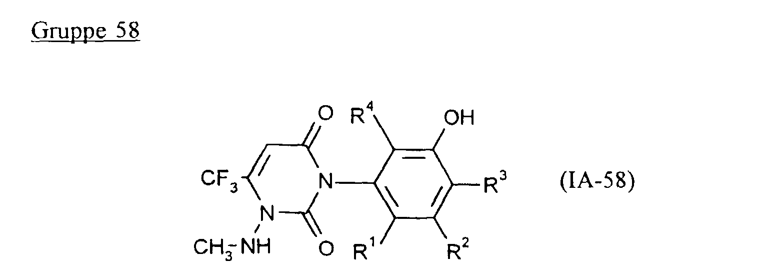 R¹, R², R³ und R⁴ haben hierbei beispielhaft die oben in Gruppe 1 angegebenen Bedeutungen. 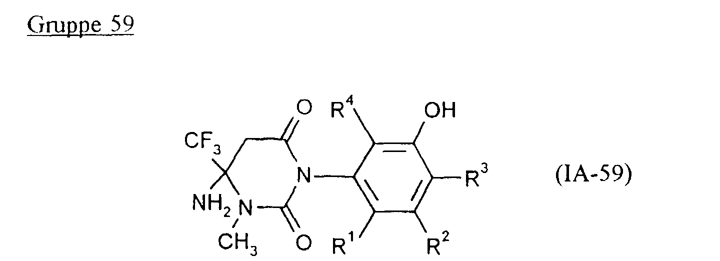 R¹, R², R³ und R⁴ haben hierbei beispielhaft die oben in Gruppe 1 angegebenen Bedeutungen. 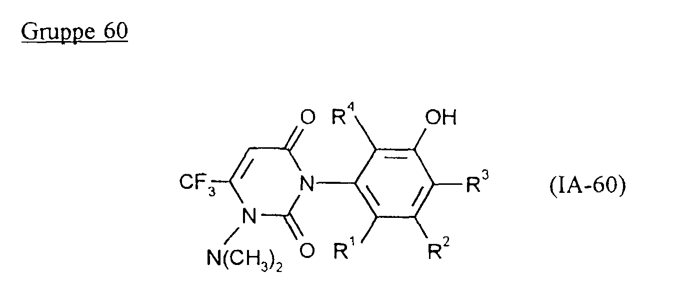 R¹, R², R³ und R⁴ haben hierbei beispielhaft die oben in Gruppe 1 angegebenen Bedeutungen. 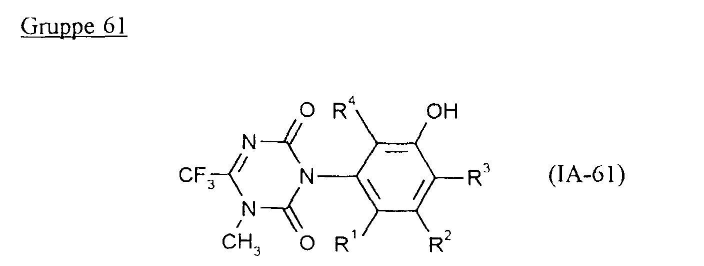 R¹, R², R³ und R⁴ haben hierbei beispielhaft die oben in Gruppe 1 angegebenen Bedeutungen. 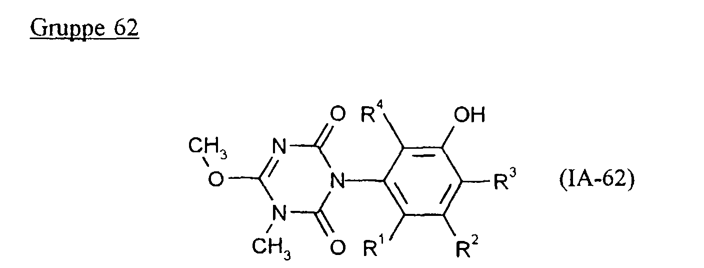 R¹, R², R³ und R⁴ haben hierbei beispielhaft die oben in Gruppe 1 angegebenen Bedeutungen. 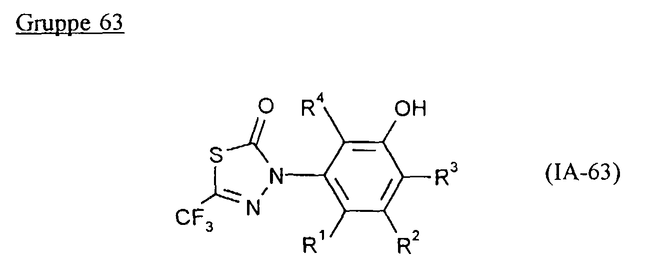 R¹, R², R³ und R⁴ haben hierbei beispielhaft die oben in Gruppe 1 angegebenen Bedeutungen. 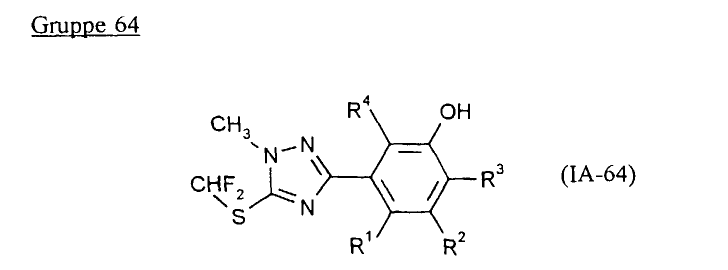 R¹, R², R³ und R⁴ haben hierbei beispielhaft die oben in Gruppe 1 angegebenen Bedeutungen. 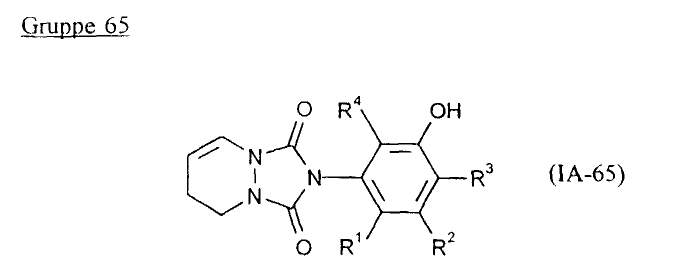 R¹, R², R³ und R⁴ haben hierbei beispielhaft die oben in Gruppe 1 angegebenen Bedeutungen. 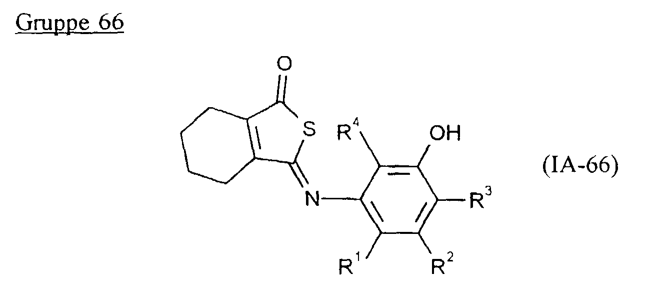 R¹, R², R³ und R⁴ haben hierbei beispielhaft die oben in Gruppe 1 angegebenen Bedeutungen. 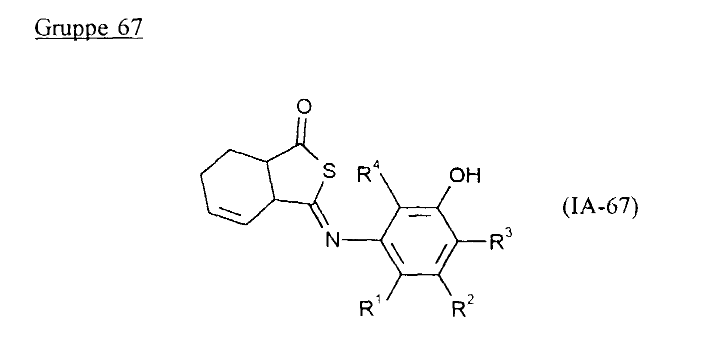 R¹, R², R³ und R⁴ haben hierbei beispielhaft die oben in Gruppe 1 angegebenen Bedeutungen. 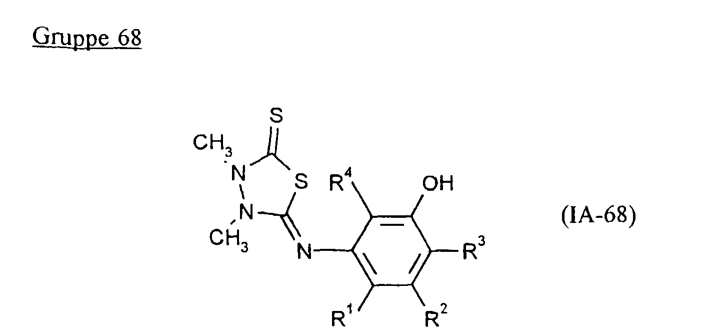 R¹, R², R³ und R⁴ haben hierbei beispielhaft die oben in Gruppe 1 angegebenen Bedeutungen. 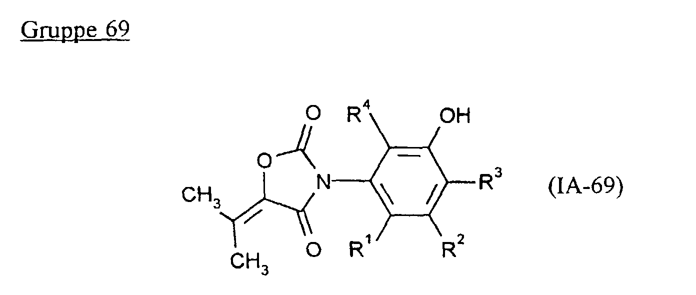 R¹, R², R³ und R⁴ haben hierbei beispielhaft die oben in Gruppe 1 angegebenen Bedeutungen. 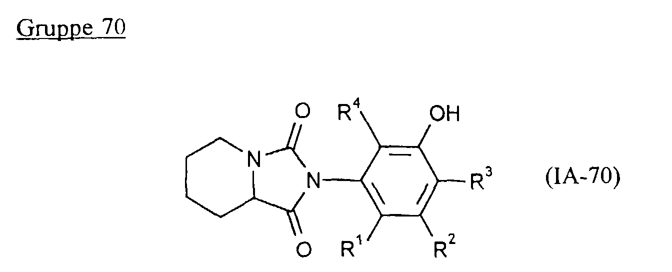 R¹, R², R³ und R⁴ haben hierbei beispielhaft die oben in Gruppe 1 angegebenen Bedeutungen. 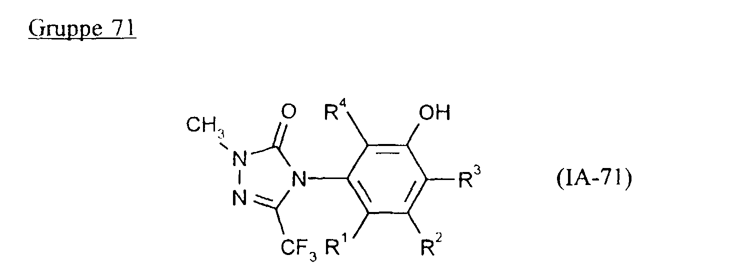 R¹, R², R³ und R⁴ haben hierbei beispielhaft die oben in Gruppe 1 angegebenen Bedeutungen.

Verwendet man beispielsweise 2-Fluor-4-trifluormethyl-benzonitril und 3-Hydroxypropionitril als Ausgangsstoffe, so kann der Reaktionsablauf beim erfindungsgemäßen Verfahren durch das folgende Formelschema skizziert werden:

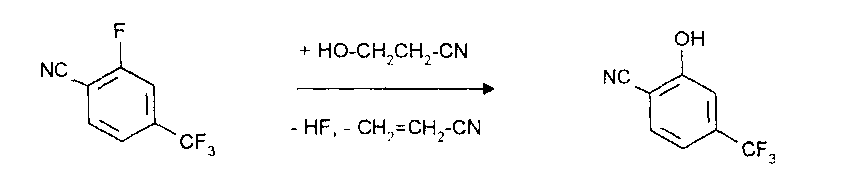

Die beim erfindungsgemäßen Verfahren zur Herstellung von Verbindungen der Formel (I) als Ausgangsstoffe zu verwendenden Halogenarene sind durch die Formel (II) allgemein definiert. In der Formel (II) haben n, R und Z vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für n, R und Z angegeben wurden; X steht vorzugsweise für Fluor, Chlor oder Brom, insbesondere für Fluor oder Chlor.

Die Ausgangsstoffe der allgemeinen Formel (II) sind bekannt und/oder können nach bekannten Verfahren hergestellt werden (vgl. EP 609734, EP 648749, EP 648772).

Das beim erfindungsgemäßen Verfahren weiter als Ausgangsverbindung zu verwendende 3-Hydroxy-propionitril der Formel (III) ist eine bekannte Synthesechemikalie.

Das erfindungsgemäße Verfahren zur Herstellung von Verbindungen der allgemeinen Formel (I) wird vorzugsweise unter Verwendung eines Reaktionshilfsmittels durchgeführt. Als Reaktionshilfsmittel kommen hierbei im allgemeinen die üblichen anorganischen oder organischen Basen oder Säureakzeptoren in Betracht. Hierzu gehören vorzugsweise Alkalimetall- oder Erdalkalimetall- -acetate, -amide, -carbonate, -hydrogencarbonate, -hydride, -hydroxide oder -alkanolate, wie beispielsweise Natrium-, Kalium- oder Calcium-acetat, Lithium-, Natrium-, Kalium- oder Calcium-amid, Natrium-, Kalium- oder Calcium-carbonat, Natrium-, Kalium- oder Calcium-hydrogencarbonat, Lithium-, Natrium-, Kalium- oder Calciumhydrid, Lithium-, Natrium-, Kalium-, Magnesium-, Calcium- oder Bariumhydroxid, Natrium- oder Kalium- -methanolat, -ethanolat, -n- oder -i-propanolat, -n-, -i-, -s- oder -t-butanolat; weiterhin auch basische organische Stickstoffverbindungen, wie beispielsweise Trimethylamin, Triethylamin, Tripropylamin, Tributylamin, Ethyl-diisopropylamin, N,N-Dimethyl-cyclohexylamin, Dicyclohexylamin, Ethyl-dicyclohexylamin, N,N-Dimethyl-anilin, N,N-Dimethyl-benzylamin, Pyridin, 2-Methyl-, 3-Methyl-, 4-Methyl-, 2,4-Dimethyl-, 2,6-Dimethyl-, 3,4-Dimethyl- und 3,5-Dimethyl-pyridin, 5-Ethyl-2-methyl-pyridin, 4-Dimethylaminopyridin, N-Methyl-piperidin, 1,4-Diazabicyclo[2,2,2]-octan (DABCO), 1,5-Diazabicyclo[4,3,0]-non-5-en (DBN), oder 1,8 Diazabicyclo[5,4,0]-undec-7-en (DBU).

Alkalimetall- oder Erdalkalimetall-hydride, wie z.B. Natriumhydrid oder Calciumhydrid, Alkalimetall- oder Erdalkalimetall-hydroxide, wie z.B. Natriumhydroxid, Kaliumhydroxid, oder Calciumhydroxid, sowie Alkalimetallalkoholate, wie z.B. Natrium-t-butylat oder Kalium-t-butylat, werden als Reaktionshilfsmittel ganz besonders bevorzugt.

Das erfindungsgemäße Verfahren zur Herstellung von Verbindungen der allgemeinen Formel (I) wird vorzugsweise unter Verwendung eines Verdünnungsmittels durchgeführt. Als Verdünnungsmittel kommen hierbei vor allem inerte organische Lösungsmittel in Betracht. Hierzu gehören insbesondere aliphatische, alicyclische oder aromatische, gegebenenfalls halogenierte Kohlenwasserstoffe, wie beispielsweise Benzin, Paraffin, Benzol, Toluol, Xylol, Chlorbenzol, Dichlorbenzol, Petrolether, Hexan, Cyclohexan, Dichlormethan, Chloroform, Tetrachlorkohlenstoff; Ether, wie Diethylether, Diisopropylether, Methyl-t-butyl-ether (MTBE), t-Amyl-methyl-ether (TAME), Dioxan, Tetrahydrofuran oder Ethylenglykoldimethyl- oder -diethylether; Ketone, wie Aceton, Butanon oder Methyl-isobutyl-keton; Nitrile, wie Acetonitril, Propionitril oder Butyronitril; Amide, wie N,N-Dimethylformamid, N,N-Dimethylacetamid, N-Methyl-formanilid, N-Methyl-pyrrolidon oder Hexamethylphosphorsäuretriamid; Ester wie Essigsäuremethylester oder Essigsäureethylester, Sulfoxide, wie Dimethylsulfoxid, oder Sulfone, wie Tetramethylensulfon.

Aprotisch polare organische Lösungsmittel, wie z.B. Methyl-t-butyl-ether (MTBE), t-Amyl-methyl-ether (TAME), Methyl-isobutyl-keton, Acetonitril, Propionitril oder Butyronitril werden als Verdünnungsmittel ganz besonders bevorzugt.

Die Reaktionstemperaturen können bei der Durchführung des erfindungsgemäßen Verfahrens in einem größeren Bereich variiert werden. Im allgemeinen arbeitet man bei Temperaturen zwischen 0°C und 10°C, vorzugsweise zwischen 10°C und 60°C.
Das erfindungsgemäße Verfahren wird im allgemeinen unter Normaldruck durchgeführt. Es ist jedoch auch möglich, das erfindungsgemäße Verfahren unter erhöhtem oder vermindertem Druck - im allgemeinen zwischen 0,1 bar und 10 bar - durchzuführen.

Zur Durchführung des erfindungsgemäßen Verfahrens setzt man pro Mol an Halogenaren der Formel (II) im allgemeinen 1,0 bis 5,0 Mol, vorzugsweise 1,1 bis 3,0 Mol 3-Hydroxy-propionitril der Formel (III) und gegebenenfalls 1,0 bis 5,0 Mol, vorzugsweise 1,1 bis 3,0 Mol an Reaktionshilfsmittel ein.

In einer bevorzugten Ausführungsform des erfindungsgemäßen Verfahrens wird das 3-Hydroxy-propionitril der Formel (III) in einem geeigneten Verdünnungsmittel vorgelegt und das Reaktionshilfsmittel wird dann dazu gegeben. Nach kurzem Rühren der Mischung bei Raumtemperatur (ca. 20°C) wird das Halogenaren der Formel (II) dazu gegeben und die Reaktionsmischung bis zum Ende der Umsetzung gerührt. Die Aufarbeitung und Isolierung der Reaktionsprodukte erfolgt nach bekannten Methoden (vgl. die Herstellungsbeispiele).

Die nach dem erfindungsgemäßen Verfahren herzustellenden Hydroxyarene sind biologisch aktive Wirkstoffe und/oder können als Zwischenprodukte zur Herstellung von biologisch aktiven Verbindungen, insbesondere von Herbiziden, verwendet werden (vgl. EP 609734, EP 648749, EP 648772).

### Herstellungsbeispiele:

### Beispiel 1

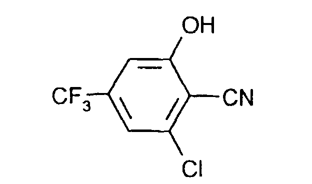

In eine Mischung aus 2,3 g (33 mMol) 3-Hydroxy-propionitril und 100 ml Acetonitril werden bei 25°C portionsweise 0,99 g (33 mMol) Natriumhydrid (80 %ig in Paraffin) eingetragen. Die Mischung wird ca. 15 Minuten bei Raumtemperatur (ca. 20°C) gerührt, anschließend mit 7,2 g (30 mol) 2,6-Dichlor-4-trifluormethyl-benzonitril versetzt und 12 Stunden bei Raumtemperatur gerührt. Die Mischung wird im Wasserstrahlvakuum eingeengt, der Rückstand in Wasser aufgenommen, mit konz. Salzsäure angesäuert und das ausgefallene Produkt durch Filtration isoliert.

Man erhält 5,6 g (84 % der Theorie) 2-Cyano-3-chlor-5-trifluormethyl-phenol vom Schmelzpunkt 138°C.

### Beispiel 2

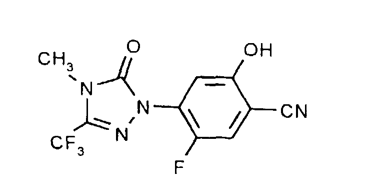

71 g (1,0 Mol) 3-Hydroxy-propionitril werden in 500 ml Acetonitril bei ca. 20°C portionsweise mit 28,8 g (1,0 Mol) Natriumhydrid (80 %ig in Paraffin) versetzt und die Mischung wird dann 15 Minuten bei 25°C gerührt. Anschließend werden 121,6 g (0,4 Mol) 2-(2,5-Difluor-4-cyano-phenyl)-4-methyl-5-trifluormethyl-2,4-dihydro-3H-1,2,4-triazol-3-on eingetragen und die Reaktionsmischung wird 12 Stunden bei ca. 25°C gerührt. Die Mischung wird dann auf Eiswasser gegeben mit konz. Salzsäure angesäuert und das ausgefallene Produkt durch Filtration isoliert.

Man erhält 114 g (94 % der Theorie) 2-(2-Fluor-4-cyano-5-hydroxy-phenyl)-4-methyl-5-trifluormethyl-2,4-dihydro-3H-1,2,4-triazol-3-on vom Schmelzpunkt 208°C.

### Beispiel 3

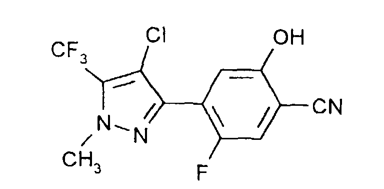

Zu einer Lösung von 11,0 g (0,15 Mol) 3-Hydroxy-propionitril in 100 ml Acetonitril gibt man 4,5 g (0,15 Mol) 80%iges Natriumhydrid in Mineralöl. Die Mischung wird ca. 15 Minuten bei Raumtemperatur (ca. 20°C) gerührt und dann mit 19,3 g (0,06 Mol) 4-Chlor-3-(4-Cyano-2,5-difluor-phenyl)-1-methyl-5-trifluormethyl-1H-pyrazol versetzt. Nach 16-stündigem Rühren bei Raumtemperatur wird die Reaktionsmischung mit Wasser und Dichlormethan versetzt, mit Salzsäure angesäuert und die organische Phase abgetrennt. Die organische Phase wird nacheinander mit gesättiger Natriumhydrogencarbonat- und Kochsalz-Lösung gewaschen, über Magnesiumsulfat getrocknet und im Wasserstrahlvakuum vom Lösungsmittel befreit. Das so erhaltene Rohprodukt wird säulenchromatographisch mit Dichlormethan als Laufmittel gereinigt.

Man erhält 6,1 g (32 % der Theorie) 4-Chlor-3-(4-cyano-2-fluor-5-hydroxyphenyl)-1-methyl-5-trifluormethyl-1H-pyrazol mit einem Schmelzpunkt von 151°C.

Analog zu den Herstellungsbeispielen 1 bis 3 und entsprechend der allgemeinen Beschreibung des erfindungsgemäßen Verfahrens können beispielsweise auch die in der Tabelle 1 aufgeführten Verbindungen der nachstehenden Formel (IB) hergestellt werden.

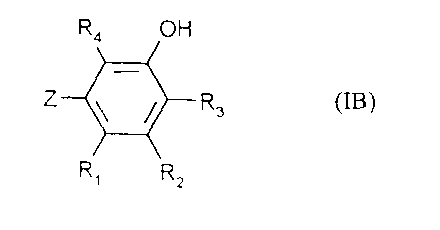

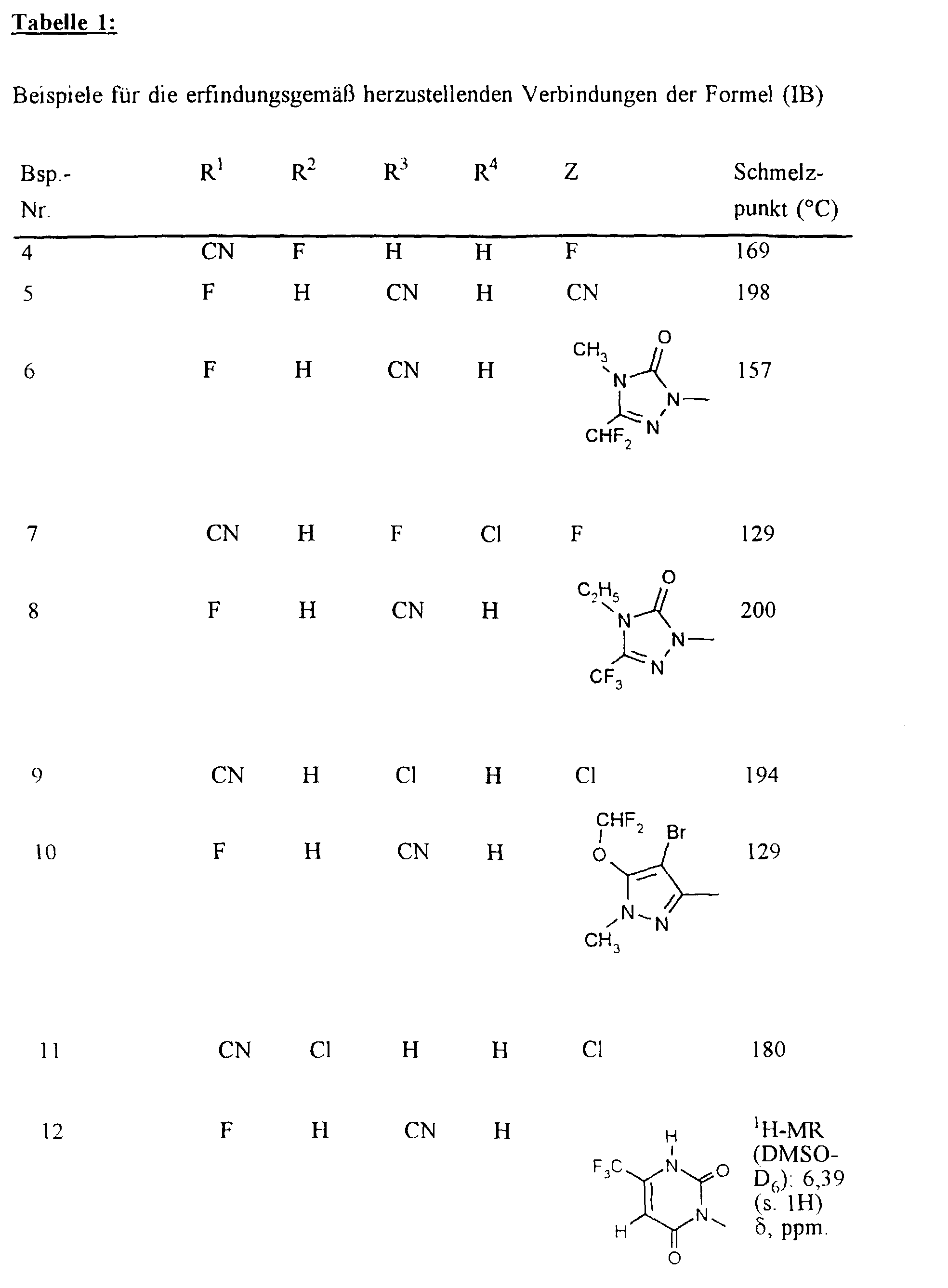

## Patentansprüche

1. Verfahren zur Herstellung von Hydroxyarenen der allgemeinen Formel (I) 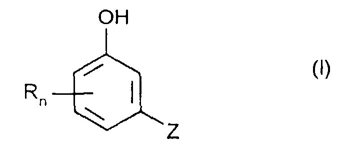 in welcher
n für die Zahlen 1, 2, 3 oder 4 steht,
R für Cyano, Carboxy, Formyl, Nitro, Halogen oder für jeweils gegebenenfalls substituiertes Alkyl, Alkoxy, Alkylthio, Alkylsulfinyl, Alkylsulfonyl, Alkylcarbonyl oder Alkoxycarbonyl steht und
Z für Wasserstoff, Cyano, Nitro, Halogen, Alkyl, Halogenalkyl, oder für jeweils gegebenenfalls substituiertes, monocyclisches oder bicyclisches, gesättigtes oder ungesättigtes Heterocyclyl, Heterocyclylamino oder Heterocyclylimino steht,
dadurch gekennzeichnet, daß man
Halogenarene der allgemeinen Formel (II) 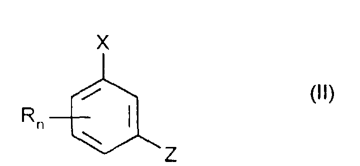 in welcher
n, R und Z die oben angegebenen Bedeutungen haben und
X für Halogen steht,
mit 3-Hydroxy-propionitril der Formel (III)
HO-CH₂CH₂-CN (III)
umsetzt.

2. Verfahren gemäß Anspruch 1, dadurch gekennzeichnet, daß man Halogenarene der Formel (II) einsetzt, in denen
n für die Zahlen 1, 2 oder 3 steht,
R für Cyano, Carboxy, Formyl, Nitro, Halogen oder für jeweils gegebenenfalls durch Cyano, Halogen, C₁-C₄-Alkoxy oder C₁-C₄-Alkylthio substituiertes Alkyl, Alkoxy, Alkylthio, Alkylsulfinyl, Alkylsulfonyl, Alkylcarbonyl oder Alkoxycarbonyl mit jeweils 1 bis 6 Kohlenstoffatomen steht,
Z für Wasserstoff, Cyano, Halogen oder für jeweils gegebenenfalls substituiertes, monocyclisches oder bicyclisches, gesättigtes oder ungesättigtes Heterocyclyl, Heterocyclylamino oder Heterocyclylimino mit jeweils 2 bis 6 Kohlenstoffatomen und 1 bis 4 Stickstoffatomen im heterocyclischen Ringsystem steht, welches gegebenenfalls zusätzlich ein Sauerstoff- oder Schwefelatom und/oder gegebenenfalls bis zu drei Gruppierungen aus der Reihe -CO-, -CS-, -SO- und/oder SO₂- enthält, und welches gegebenenfalls substituiert ist durch eine oder mehrere Gruppierungen aus der Reihe Nitro, Hydroxy, Amino, Cyano, Carboxy, Carbamoyl, Thiocarbamoyl, Halogen, C₁-C₆-Alkyl (welches gegebenenfalls durch Halogen oder C₁-C₄-Alkoxy substituiert ist), C₂-C₆-Alkenyl oder C₂-C₆-Alkinyl (welche jeweils gegebenenfalls durch Halogen substituiert sind), C₁-C₆-Alkoxy oder C₁-C₆-Alkoxy-carbonyl (welche jeweils gegebenenfalls durch Halogen oder C₁-C₄-Alkoxy substituiert sind), C₂-C₆-Alkenyloxy oder C₂-C₆-Alkinyloxy (welche jeweils gegebenenfalls durch Halogen substituiert sind), C₁-C₆-Alkylthio, C₂-C₆-Alkenylthio oder C₂-C₆-Alkinylthio (welche jeweils gegebenenfalls durch Halogen substituiert sind), C₁-C₆-Alkylamino oder Di-(C₁-C₄-alkyl)-amino, C₃-C₆-Cycloalkyl oder C₃-C₆-Cycloalkyl-C₁-C₄-alkyl (welche jeweils gegebenenfalls durch Halogen und/oder C₁-C₄-Alkyl substituiert sind), Phenyl, Phenoxy, Phenylthio, Phenylsulfinyl, Phenylsulfonyl oder Phenylamino (welche jeweils gegebenenfalls durch Nitro, Cyano, Halogen, C₁-C₄-Alkyl, C₁-C₄-Halogenalkyl, C₁-C₄-Alkyloxy, C₁-C₄-Halogenalkyloxy und/oder C₁-C₄-Alkoxy-carbonyl substituiert sind), und
X für Fluor, Chlor oder Brom steht.

3. Verfahren gemäß Anspruch 1, dadurch gekennzeichnet, daß man Halogenarene der Formel (II) einsetzt, in denen
n für die Zahlen 1 oder 2 steht,
R für Cyano, Carboxy, Formyl, Nitro, Halogen oder für jeweils gegebenenfalls durch Cyano, Halogen, Methoxy, Ethoxy, Methylthio oder Ethylthio substituiertes Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl, Ethylsulfonyl, Acetyl, Propionyl, Methoxycarbonyl oder Ethoxycarbonyl steht,
Z für Wasserstoff, Cyano, Halogen oder für jeweils gegebenenfalls substituiertes, monocyclisches ode bicyclisches, gesättigtes oder ungesättigtes Heterocyclyl, Heterocyclylamino oder Heterocyclylimino mit jeweils 2 bis 6 Kohlenstoffatomen und 1 bis 4 Stickstoffatomen im heterocyclischen Ringsystem steht, welches gegebenenfalls zusätzlich ein Sauerstoff- oder Schwefelatom und/oder gegebenenfalls bis zu zwei Gruppierungen aus der Reihe -CO-, -CS-, -SO- und/oder SO₂- enthält, und welches gegebenenfalls substituiert ist durch eine oder mehrere Gruppierungen aus der Reihe Nitro, Hydroxy, Amino, Cyano, Carboxy, Carbamoyl, Thiocarbamoyl, Fluor, Chlor, Brom; Methyl, Ethyl, n- oder i-Propyl, n-, i-, s- oder t-Butyl, (welche gegebenenfalls durch Fluor, Chlor, Methoxy oder Ethoxy substituiert sind); Propenyl, Butenyl, Propinyl oder Butinyl (welche jeweils gegebenenfalls durch Fluor oder Chlor substituiert sind); Methoxy, Ethoxy, n- oder i-Propoxy, n-, i-, s- oder t-Butoxy, Methoxycarbonyl oder Ethoxycarbonyl (welche jeweils gegebenenfalls durch Fluor, Chlor, Methoxy oder Ethoxy substituiert sind); Propenyloxy, Butenyloxy, Propinyloxy oder Butinyloxy (welche gegebenenfalls durch Fluor oder Chlor substituiert sind); Methylthio, Ethylthio, n- oder i-Propylthio, n-, i-, s- oder t-Butylthio, Propenylthio, Butenylthio, Propinylthio oder Butinylthio (welche jeweils gegebenenfalls durch Fluor oder Chlor substituiert sind); Methylamino, Ethylamino, n- oder i-Propylamino, n-, i-, s- oder t-Butylamino, Dimethylamino oder Diethylamino; Cyclopropyl, Cyclobutyl, Cyclopentyl, Cyclohexyl, Cyclopropylmethyl, Cyclobutylmethyl, Cyclopentylmethyl oder Cyclohexylmethyl (welche jeweils gegebenenfalls durch Fluor, Chlor, Methyl, Ethyl, n- oder i-Propyl substituiert sind), Phenyl, Phenoxy, Phenylthio, Phenylsulfinyl, Phenylsulfonyl oder Phenylamino (welche jeweils gegebenenfalls durch Nitro, Cyano, Fluor, Chlor, Brom, Methyl, Ethyl, n- oder i-Propyl, Trifluormethyl, Methoxy, Ethoxy, n- oder i-Propoxy, Difluormethoxy, Trifluormethoxy, Methoxycarbonyl oder Ethoxycarbonyl substituiert sind), und
X für Fluor oder Chlor steht.

4. Verfahren gemäß Ansprüchen 1 bis 3, dadurch gekennzeichnet, daß dieses in Gegenwart eines Reaktionshilfsmittels durchgeführt wird.

5. Verfahren gemäß Ansprüchen 1 bis 3, dadurch gekennzeichnet, daß dieses in Gegenwart eines Verdünnungsmittels durchgeführt wird.

6. Verfahren gemäß Ansprüchen 1 bis 3, dadurch gekennzeichnet, daß dieses bei Temperaturen zwischen 0 und 100°C durchgeführt wird.

## Claims

1. Process for preparing hydroxyarenes of the general formula (I) 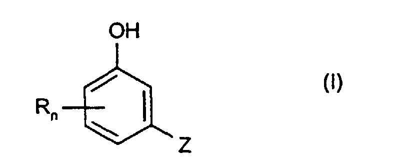 in which
n represents the numbers 1, 2, 3 or 4,
R represents cyano, carboxyl, formyl, nitro, halogen or represents alkyl, alkoxy, alkylthio, alkylsulphinyl, alkylsulphonyl, alkylcarbonyl or alkoxycarbonyl, each of which is optionally substituted, and
Z represents hydrogen, cyano, nitro, halogen, alkyl, halogenoalkyl, or represents monocyclic or bicyclic, saturated or unsaturated heterocyclyl, heterocyclylamino or heterocyclylimino, each of which is optionally substituted,
characterized in that
halogenoarenes of the general formula (II) 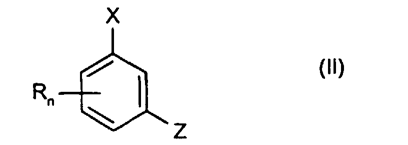 in which
n, R and Z are each as defined above and
x represents halogen
are reacted with 3-hydroxy-propionitrile of the formula (III)
HO-CH₂CH₂-CN (III).

2. Process according to Claim 1, characterized in that halogenoarenes of the formula (II) are used in which
n represents the numbers 1, 2 or 3,
R represents cyano, carboxyl, formyl, nitro, halogen or represents alkyl, alkoxy, alkylthio, alkylsulphinyl, alkylsulphonyl, alkylcarbonyl or alkoxycarbonyl having in each case 1 to 6 carbon atoms and being in each case optionally substituted by cyano, halogen, C₁-C₄-alkoxy or C₁-C₄-alkylthio,
Z represents hydrogen, cyano, halogen or represents monocyclic or bicyclic, saturated or unsaturated heterocyclyl, heterocyclylamino or heterocyclylimino having in each case 2 to 6 carbon atoms and 1 to 4 nitrogen atoms in the heterocyclic ring system and being in each case optionally substituted, which optionally additionally contains an oxygen or sulphur atom and/or optionally up to three groups selected from the series -CO-, -CS-, -SO- and/or SO₂-, and which is optionally substituted by one or more groups selected from the series nitro, hydroxyl, amino, cyano, carboxyl, carbamoyl, thiocarbamoyl, halogen, C₁-C₆-alkyl (which is optionally substituted by halogen or C₁-C₄-alkoxy), C₂-C₆-alkenyl or C₂-C₆-alkinyl (which are in each case optionally substituted by halogen), C₁-C₆-alkoxy or C₁-C₆-alkoxy-carbonyl (which are in each case optionally substituted by halogen or C₁-C₄-alkoxy), C₂-C₆-alkenyloxy or C₂-C₆-alkinyloxy (which are in each case optionally substituted by halogen), C₁-C₆-alkylthio, C₂-C₆-alkenylthio or C₂-C₆-alkinylthio (which are in each case optionally substituted by halogen), C₁-C₆-alkylamino or di-(C₁-C₄-alkyl) -amino, C₃-C₆-cycloalkyl or C₃-C₆-cycloalkyl-C₁-C₄-alkyl (which are in each case optionally substituted by halogen and/or C₁-C₄-alkyl), phenyl, phenoxy, phenylthio, phenylsulphinyl, phenylsulphonyl or phenylamino (which are in each case optionally substituted by nitro, cyano, halogen, C₁-C₄-alkyl, C₁-C₄-halogenoalkyl, C₁-C₄-alkyloxy, C₁-C₄-halogenoalkyloxy and/or C₁-C₄-alkoxy-carbonyl), and
X represents fluorine, chlorine or bromine.

3. Process according to Claim 1, characterized in that halogenoarenes of the formula (II) are used in which
n represents the numbers 1 or 2,
R represents cyano, carboxyl, formyl, nitro, halogen or represents methyl, ethyl, n- or i-propyl, n-, i-, s- or t-butyl, methoxy, ethoxy, n- or i-propoxy, methylthio, ethylthio, n- or i-propylthio, methylsulphinyl, ethylsulphinyl, methylsulphonyl, ethylsulphonyl, acetyl, propionyl, methoxycarbonyl or ethoxycarbonyl, each of which is optionally substituted by cyano, halogen, methoxy, ethoxy, methylthio or ethylthio,
Z represents hydrogen, cyano, halogen or represents monocyclic or bicyclic, saturated or unsaturated heterocyclyl, heterocyclylamino or heterocyclylimino having in each case 2 to 6 carbon atoms and 1 to 4 nitrogen atoms in the heterocyclic ring system and being in each case optionally substituted, which optionally additionally contains an oxygen or sulphur atom and/or optionally up to two groups selected from the series -CO-, -CS-, -SO- and/or SO₂-, and which is optionally substituted by one or more groups selected from the series nitro, hydroxyl, amino, cyano, carboxyl, carbamoyl, thiocarbamoyl, fluorine, chlorine, bromine; methyl, ethyl, n- or i-propyl, n-, i-, s- or t-butyl (which are optionally substituted by fluorine, chlorine, methoxy or ethoxy); propenyl, butenyl, propinyl or butinyl (which are in each case optionally substituted by fluorine or chlorine); methoxy, ethoxy, n- or i-propoxy, n-, i-, s- or t-butoxy, methoxycarbonyl or ethoxycarbonyl (which are in each case optionally substituted by fluorine, chlorine, methoxy or ethoxy); propenyloxy, butenyloxy, propinyloxy or butinyloxy (which are optionally substituted by fluorine or chlorine); methylthio, ethylthio, nor i-propylthio, n-, i-, s- or t-butylthio, propenylthio, butenylthio, propinylthio or butinylthio (which are in each case optionally substituted by fluorine or chlorine); methylamino, ethylamino, n- or i-propylamino, n-, i-, s- or t-butylamino, dimethylamino or diethylamino; cyclopropyl, cyclobutyl, cyclopentyl, cyclohexyl, cyclopropylmethyl, cyclobutylmethyl, cyclopentylmethyl or cyclohexylmethyl (which are in each case optionally substituted by fluorine, chlorine, methyl, ethyl, n- or i-propyl), phenyl, phenoxy; phenylthio, phenylsulphinyl, phenylsulphonyl or phenylamino (which are in each case optionally substituted by nitro, cyano, fluorine, chlorine, bromine, methyl, ethyl, n- or i-propyl, trifluoromethyl, methoxy, ethoxy, n- or i-propoxy, difluoromethoxy, trifluoromethoxy, methoxycarbonyl or ethoxycarbonyl), and
X represents fluorine or chlorine.

4. Process according to any of Claims 1 to 3, characterized in that this process is carried out in the presence of a reaction auxiliary.

5. Process according to any of Claims 1 to 3, characterized in that this process is carried out in the presence of a diluent.

6. Process according to any of Claims 1 to 3, characterized in that this process is carried out at temperatures between 0 and 100°C.

## Revendications

1. Procédé de production d'hydroxyarènes de formule générale (I) 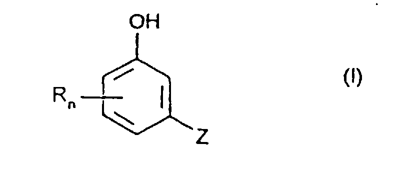 dans laquelle
n représente les nombres 1, 2, 3 ou 4,
R est un groupe cyano, carboxy, formyle, nitro, un halogène, ou bien un groupe alkyle, alkoxy, alkylthio, alkylsulfinyle, alkylsulfonyle, alkylcarbonyle ou alkoxycarbonyle dont chacun est éventuellement substitué, et
Z est l'hydrogène, un groupe cyano, nitro, un halogène, un groupe alkyle, halogénalkyle, ou bien un groupe hétérocyclyle, hétérocyclylamino ou hétérocyclylimino saturé ou non saturé, monocyclique ou bicyclique, dont chacun est éventuellement substitué,
caractérisé en ce qu'on fait réagir des arènes halogénés de formule générale (II) 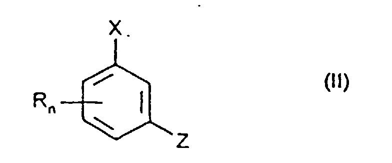 dans laquelle
n, R et Z ont les définitions indiquées ci-dessus et
X désigne un halogène,
avec le 3-hydroxy-propionitrile de formule (III)
HO-CH₂-CH₂-CN (III)

2. Procédé suivant la revendication 1, caractérisé en ce qu'on utilise des arènes halogénés de formule (II), dans lesquels
n représente les nombres 1, 2 ou 3,
R est un groupe cyano, carboxy, formyle, nitro, un halogène ou un groupe alkyle, alkoxy, alkylthio, alkylsulfinyle, alkylsulfonyle, alkylcarbonyle ou alkoxycarbonyle ayant chacun 1 à 6 atomes de carbone et dont chacun est éventuellement substitué par un radical cyano, halogéno, alkoxy en C₁ à C₄ ou alkylthio en C₁ à C₄,
Z représente l'hydrogène, un groupe cyano, un halogène ou un groupe hétérocyclyle, hétérocyclylamino ou hétérocyclylimino saturé ou non saturé, monocyclique ou bicyclique, éventuellement substitué dans chaque cas, ayant chacun 2 à 6 atomes de carbone et 1 à 4 atomes d'azote dans le noyau hétérocyclique, qui contient en outre le cas échéant un atome d'oxygène ou de soufre et/ou éventuellement jusqu'à trois groupements de la série -CO-, -CS-, -SO- et/ou SO₂-, et qui est éventuellement substitué par un ou plusieurs groupements de la série nitro, hydroxy, amino, cyano, carboxy, carbamoyle, thiocarbamoyle, halogéno, alkyle en C₁ à C₆ (qui est éventuellement substitué par un halogène ou un radical alkoxy en C₁ à C₄), alcényle en C₂ à C₆ ou alcynyle en C₂ à C₆ (qui sont chacun éventuellement substitués par un halogène), alkoxy en C₁ à C₆ ou (alkoxy en C₁ à C₆)carbonyle (qui sont chacun éventuellement substitués par un halogène ou un radical alkoxy en C₁ à C₄), alcényloxy en C₂ à C₆ ou alcynyloxy en C₂ à C₆ (qui sont chacun éventuellement substitués par un halogène), alkylthio en C₁ à C₆, alcénylthio en C₂ à C₆ ou alcynylthio en C₂ à C₆ (qui sont chacun éventuellement substitués par un halogène), alkylamino en C₁ à C₆ ou di(alkyle en C₁ à C₄)amino, cycloalkyle en C₃ à C₆ ou (cycloalkyle en C₃ à C₆)-(alkyle en C₁ à C₄) (qui sont chacun éventuellement substitués par un halogène et/ou un radical alkyle en C₁ à C₄), phényle, phénoxy, phénylthio, phénylsulfinyle, phénylsulfonyle ou phénylamino (qui sont substitués chacun le cas échéant par un radical nitro, cyano, halogéno, alkyle en C₁ à C₄, halogénalkyle en C₁ à C₄, alkyloxy en C₁ à C₄, halogénalkyloxy en C₁ à C₄ et/ou (alkoxy en C₁ à C₄) -carbonyle),
et
X représente le fluor, le chlore ou le brome.

3. Procédé suivant la revendication 1, caractérisé en ce qu'on utilise des arènes halogénés de formule (II), dans lesquels
n représente les nombres 1 ou 2,
R est un groupe cyano, carboxy, formyle, nitro, un halogène ou un groupe méthyle, éthyle, n-propyle, isopropyle, n-butyle, isobutyle, sec.butyle, tertiobutyle, méthoxy, éthoxy, n-propoxy, isopropoxy, méthylthio, éthyltio, n-propylthio, isopropylthio, méthylsulfinyle, éthylsulfinyle, méthylsulfonyle, éthylsulfonyle, acétyle, propionyle, méthoxycarbonyle ou éthoxycarbonyle dont chacun est substitué le cas échéant par un radical cyano, halogéno, méthoxy, éthoxy, méthylthio ou éthylthio,
Z représente l'hydrogène, un groupe cyano, un halogène ou un groupe hétérocyclyle, hétérocyclylamino ou hétérocyclylimino saturé ou non saturé, monocyclique ou bicyclique, éventuellement substitué dans chaque cas, ayant chacun 2 à 6 atomes de carbone et 1 à 4 atomes d'azote dans le noyau hétérocyclique, qui contient en outre le cas échéant un atome d'oxygène ou de soufre et/ou, le cas échéant, jusqu'à deux groupements de la série -CO-, -CS-, -SO- et/ou SO₂-, et qui est substitué le cas échéant par un ou plusieurs groupements de la série nitro, hydroxy, amino, cyano, carboxy, carbamoyle, thiocarbamoyle, fluoro, chloro, bromo, méthyle, éthyle, n-propyle, isopropyle, n-butyle, isobutyle, sec.-butyle, tertiobutyle (qui sont substitués le cas échéant par un radical fluoro, chloro, méthoxy ou éthoxy), propényle, butényle, propynyle ou butynyle (qui sont substitués chacun le cas échéant par du fluor ou du chlore), méthoxy, éthoxy, n-propoxy, isopropoxy, n-butoxy, isobutoxy, sec.-butoxy, tertio-butoxy, méthoxycarbonyle ou éthoxycarbonyle (qui sont substitués chacun le cas échéant par un radical fluoro, chloro, méthoxy ou éthoxy), propényloxy, butényloxy, propynyloxy ou butynyloxy (qui sont éventuellement substitués par du fluor ou du chlore), méthylthio, éthylthio, n-propylthio, isopropylthio, n-butylthio, isobutylthio, sec.-butylthio, tertio-butylthio, propénylthio, buténylthio, propynylthio ou butynylthio (qui sont éventuellement substitués dans chaque cas par du fluor ou du chlore), méthylamino, éthylamino, n-propylamino, isopropylamino, n-butylamino, isobutylamino, sec.-butylamino, tertio-butylamino, diméthylamino ou diéthylamino ; cyclopropyle, cyclobutyle, cyclopentyle, cyclohexyle, cyclopropylméthyle, cyclobutylméthyle, cyclopentyl-méthyle ou cyclohexylméthyle (qui sont substitués chacun le cas échéant par un radical fluoro, chloro, méthyle, éthyle, n-propyle ou isopropyle), phényle, phénoxy, phénylthio, phénylsulfinyle, phénylsulfonyle ou phénylamino (qui sont substitués chacun le cas échéant par un radical nitro, cyano, fluoro, chloro, bromo, méthyle, éthyle, n-propyle, isopropyle, trifluorométhyle, méthoxy, éthoxy, n-propoxy, isopropoxy, difluorométhoxy, trifluorométhoxy, méthoxycarbonyle ou éthoxycarbonyle), et
X représente le fluor ou le chlore.

4. Procédé suivant les revendications 1 à 3, caractérisé en ce qu'il est mis en oeuvre en présence d'un auxiliaire de réaction.

5. Procédé suivant les revendications 1 à 3, caractérisé en ce qu'il est mis en oeuvre en présence d'un diluant.

6. Procédé suivant les revendications 1 à 3, caractérisé en ce qu'il est mis en oeuvre à des températures comprises entre 0 et 100°C.
